(19) Europäisches Patentamt
European Patent Office
Office européen des brevets

(11) EP 1 315 720 B1

(12) **EUROPÄISCHE PATENTSCHRIFT**

(45) Veröffentlichungstag und Bekanntmachung des Hinweises auf die Patenterteilung:
**06.07.2005 Patentblatt 2005/27**

(21) Anmeldenummer: **01976108.9**

(22) Anmeldetag: **18.08.2001**

(51) Int Cl.[7]: **C07D 413/12**, C07D 498/04, A61K 31/5375, A61P 35/00

(86) Internationale Anmeldenummer:
**PCT/EP2001/009536**

(87) Internationale Veröffentlichungsnummer:
**WO 2002/018376 (07.03.2002 Gazette 2002/10)**

(54) **BICYCLISCHE HETEROCYCLEN, DIESE VERBINDUNGEN ENTHALTENDE ARZNEIMITTEL, DEREN VERWENDUNG UND VERFAHREN ZU IHRER HERSTELLUNG**

BICYCLIC HETEROCYCLES, MEDICAMENTS CONTAINING THESE COMPOUNDS, THEIR USE, AND METHODS FOR THE PRODUCTION THEREOF

HETEROCYCLES BICYCLIQUES, MEDICAMENTS CONTENANT LESDITS COMPOSES, LEUR UTILISATION ET PROCEDES PERMETTANT DE LES PRODUIRE

(84) Benannte Vertragsstaaten:
**AT BE CH CY DE DK ES FI FR GB GR IE IT LI LU MC NL PT SE TR**

(30) Priorität: **26.08.2000 DE 10042062**

(43) Veröffentlichungstag der Anmeldung:
**04.06.2003 Patentblatt 2003/23**

(73) Patentinhaber: **BOEHRINGER INGELHEIM PHARMA GMBH & CO. KG**
**55216 Ingelheim am Rhein (DE)**

(72) Erfinder:
- **HIMMELSBACH, Frank**
  **88441 Mittelbiberach (DE)**
- **LANGKOPF, Elke**
  **88447 Warthausen (DE)**
- **JUNG, Birgit**
  **55270 Schwabenhein (DE)**
- **BLECH, Stefan**
  **88447 Warthausen (DE)**
- **SOLCA, Flavio**
  **A-1230 Wien (AT)**

(56) Entgegenhaltungen:
**WO-A-00/51991** **WO-A-97/38983**
**WO-A-99/09016** **US-A- 6 002 008**

- **TSOU H-R ET AL: "6-SUBSTITUTED-4-(3-BROMOPHENYLAMINO) QUINA ZOLINES AS PUTATIVE IRREVERSIBLE INHIBITORS OF THE EPIDERMAL GROWTH FACTOR RECEPTOR (EGFR) AND HUMAN EPIDERMAL GROWTH FACTOR RECEPTOR (HER-2) TYROSINE KINASES WITH ENHANCED ANTITUMOR ACTIVITY" JOURNAL OF MEDICINAL CHEMISTRY, AMERICAN CHEMICAL SOCIETY, US, Bd. 44, Nr. 17, 2001, Seiten 2719-2734, XP001026039 ISSN: 0022-2623**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 315 720 B1

Printed by Jouve, 75001 PARIS (FR)

## Beschreibung

Gegenstand der vorliegenden Erfindung sind bicyclische Heterocyclen der allgemeinen Formel

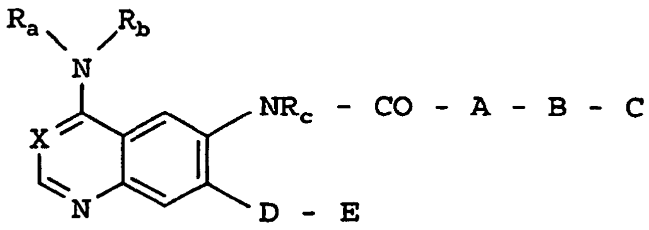

, (I)

deren Tautomeren, deren Stereoisomere und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung.

In der WO 00/51991 werden 4-Amino-chinazolin- Derivate beschrieben, welche eine Hemmwirkung auf die durch Tyrosinkinasen vermittelte Signaltransduktion aufweisen.

In der obigen allgemeinen Formel I bedeutet

$R_a$ ein Wasserstoffatom oder eine Methylgruppe,

$R_b$ eine Phenyl-, Benzyl- oder 1-Phenylethylgruppe, in denen der Phenylkern jeweils durch die Reste $R_1$ bis $R_3$ substituiert ist, wobei

$R_1$ und $R_2$, die gleich oder verschieden sein können, jeweils ein Wasserstoff-, Fluor-, Chlor-, Brom- oder Jodatom,

eine Methyl-, Ethyl-, Hydroxy-, Methoxy-, Ethoxy-, Amino-, Cyan-, Vinyl- oder Ethinylgruppe,

eine Aryl-, Aryloxy-, Arylmethyl- oder Arylmethoxygruppe,

eine durch 1 bis 3 Fluoratome substituierte Methyl- oder Methoxygruppe oder

$R_1$ zusammen mit $R_2$, sofern diese an benachbarte Kohlenstoffatome gebunden sind, eine -CH=CH-CH=CH-, -CH=CH-NH-oder -CH=N-NH-Gruppe und

$R_3$ ein Wasserstoff-, Fluor-, Chlor- oder Bromatom,

$R_c$ ein Wasserstoffatom oder eine Methylgruppe,

X eine durch eine Cyangruppe substituierte Methingruppe oder ein Stickstoffatom,

A eine 1,1- oder 1,2-Vinylengruppe, die jeweils durch eine oder zwei Methylgruppen oder durch eine Trifluormethylgruppe substituiert sein kann,

eine Ethinylengruppe oder

eine gegebenenfalls durch eine Methyl- oder Trifluormethylgruppe substituierte 1,3-Butadien-1,4-ylengruppe,

B eine Alkylen- oder -CO-alkylen-gruppe, in denen der Alkylenteil jeweils 1 bis 4 Kohlenstoffatome enthält, wobei die Verknüpfung der -CO-alkylengruppe mit der benachbarten Gruppe A jeweils über die Carbonylgruppe erfolgen muß,

eine -CO-O-alkylen- oder -CO-$NR_4$-alkylen-Gruppe, in denen der Alkylenteil jeweils 1 bis 4 Kohlenstoffatome enthält, wobei die Verknüpfung mit der benachbarten Gruppe A jeweils über die Carbonylgruppe erfolgen muß, in der

$R_4$ ein Wasserstoffatom oder eine Methyl- oder Ethylgruppe darstellt, oder eine Carbonylgruppe,

C eine durch den Rest $R_5$ oder durch den Rest $R_5$ und eine $C_{1-4}$-Alkylgruppe substituierte 2-Oxo-morpholin-4-yl-Gruppe, wobei

$R_5$ eine $C_{3-4}$-Alkyl-, Hydroxy-$C_{1-4}$-alkyl-, $C_{1-4}$-Alkoxy-$C_{1-4}$-alkyl-, Di-($C_{1-4}$-Alkyl)-amino-$C_{1-4}$-alkyl-, Pyrrolidino-$C_{1-4}$-alkyl-, Piperidino-$C_{1-4}$-alkyl-, Morpholino-$C_{1-4}$-alkyl-, 4- ($C_{1-4}$-Alkyl) -piperazino-$C_{1-4}$-alkyl-, $C_{1-4}$-Alkylsulfanyl-$C_{1-4}$-alkyl-, $C_{1-4}$-Alkylsulfinyl-$C_{1-4}$-alkyl-, $C_{1-4}$-Alkylsulfonyl-$C_{1-4}$-alkyl-, Cyan-$C_{1-4}$-alkyl-, $C_{1-4}$-Alkoxycarbonyl-$C_{1-4}$-alkyl-, Aminocarbonyl-$C_{1-4}$-alkyl-, $C_{1-4}$-Alkyl-aminocarbonyl-$C_{1-4}$-alkyl-, Di-($C_{1-4}$-alkyl)aminocarbonyl-$C_{1-4}$-alkyl-, Pyrrolidinocarbonyl-$C_{1-4}$-alkyl-, Piperidinocarbonyl-$C_{1-4}$-alkyl-, Morpholinocarbonyl-$C_{1-4}$-alkyl- oder eine 4-($C_{1-4}$-Alkyl)-piperazinocarbonyl-$C_{1-4}$-alkylgruppe darstellt,

eine durch zwei Reste $R_5$ substituierte 2-Oxo-morpholin-4-yl-Gruppe, wobei $R_5$ wie vorstehend erwähnt definiert ist und die beiden Reste $R_5$ gleich oder verschieden sein können,

eine 2-Oxo-morpholin-4-yl-Gruppe, in der die beiden Wasserstoffatome einer Methylengruppe durch eine gegebenenfalls durch eine oder zwei $C_{1-2}$-Alkylgruppen substituierte $-(CH_2)_m-$, $-CH_2-Y-CH_2$ -, $-CH_2-Y-CH_2-CH_2-$, $-CH_2CH_2-Y-CH_2CH_2-$ oder $-CH_2CH_2-Y-CH_2CH_2CH_2-$Brücke ersetzt sind, wobei

m die Zahl 2, 3, 4, 5 oder 6 und
Y ein Sauerstoff- oder Schwefelatom, eine Sulfinyl-, Sulfonyl- oder $C_{1-4}$-Alkylimino-Gruppe darstellt,

eine 2-Oxo-morpholin-4-yl-Gruppe, in der ein Wasserstoffatom in 5-Stellung zusammen mit einem Wasserstoffatom in 6-Stellung durch eine $-(CH_2)_n-$, $-CH_2-Y-CH_2-$, $-CH_2-Y-CH_2CH_2-$ oder $-CH_2CH_2-Y-CH_2-$Brücke ersetzt ist, wobei

Y wie vorstehend erwähnt definiert ist und
n die Zahl 2, 3 oder 4 darstellt,

oder, falls D zusammen mit E eine Gruppe $R_d$ darstellt, auch eine 2-Oxo-morpholin-4-yl-Gruppe, die durch 1 bis 4 $C_{1-2}$-Alkylgruppen substituiert sein kann,

D eine -O-$C_{1-6}$-Alkylengruppe, wobei der Alkylenteil mit dem Rest E verknüpft ist, oder

ein Sauerstoffatom, wobei dieses nicht mit einem Stickstoffatom des Restes E verknüpft sein kann, und

E eine durch 2 $C_{1-4}$-Alkylgruppen substituierte Aminogruppe, in der die Alkylreste gleich oder verschieden sein können und jeder Alkylteil ab Position 2 durch eine $C_{1-4}$-Alkoxy-, oder Di-($C_{1-4}$-Alkyl)-aminogruppe oder durch eine 4- bis 7-gliedrige Alkyleniminogruppe substituiert sein kann, wobei in den vorstehend erwähnten 6- bis 7-gliedrigen Alkyleniminogruppen jeweils eine Methylengruppe in 4-Stellung durch ein Sauerstoff-oder Schwefelatom, durch eine Sulfinyl-, Sulfonyl- oder N-($C_{1-4}$-Alkyl)-iminogruppe ersetzt sein kann,

eine gegebenenfalls durch 1 bis 4 Methylgruppen substituierte 4- bis 7-gliedrige Alkyleniminogruppe,

eine gegebenenfalls durch 1 oder 2 Methylgruppen substituierte 6- bis 7-gliedrige Alkyleniminogruppe, in der jeweils eine Methylengruppe in 4-Stellung durch ein Sauerstoff- oder Schwefelatom, durch eine Sulfinyl-, Sulfonyl- oder N-($C_{1-4}$-Alkyl)-iminogruppe ersetzt ist,

eine gegebenenfalls durch 1 bis 3 Methylgruppen substituierte Imidazolylgruppe,

eine $C_{5-7}$-Cycloalkylgruppe, in der eine Methylengruppe durch ein Sauerstoff- oder Schwefelatom, durch eine Sulfinyl-, Sulfonyl-oder N-($C_{1-4}$-Alkyl)-iminogruppe ersetzt ist, oder

D zusammen mit E ein Wasserstoffatom,

eine gegebenenfalls ab Position 2 durch eine Hydroxy- oder $C_{1-4}$-Alkoxygruppe substituierte $C_{1-6}$-Alkoxygruppe,

eine $C_{3-7}$-Cycloalkoxy- oder $C_{3-7}$-Cycloalkyl-$C_{1-4}$-alkoxygruppe,

oder einen Rest $R_d$, wobei

$R_d$ eine $C_{2-6}$-Alkoxygruppe, die ab Position 2 durch eine $C_{4-7}$-Cycloalkoxy- oder $C_{3-7}$-Cycloalkyl-$C_{1-3}$-alkoxygruppe substituiert ist,

eine $C_{4-7}$-Cycloalkoxy- oder $C_{3-7}$-Cycloalkyl-$C_{1-6}$-alkoxygruppe, in denen der Cycloalkylteil jeweils durch eine $C_{1-4}$-Alkyl-, $C_{1-4}$-Alkoxy-, Di-($C_{1-4}$-alkyl)-amino-, Pyrrolidino-, Piperidino-, Morpholino-, Piperazino-, 4-($C_{1-2}$-Alkyl)-piperazino-, $C_{1-4}$-Alkoxy-$C_{1-2}$-alkyl-, Di-($C_{1-4}$-alkyl)-amino-$C_{1-2}$-alkyl-, Pyrrolidino-$C_{1-2}$-alkyl-, Piperidino-$C_{1-2}$-alkyl-, Morpholino-$C_{1-2}$-alkyl-, Piperazino-$C_{1-2}$-alkyl- oder 4- ($C_{1-2}$-Alkyl) -piperazino-$C_{1-2}$-alkylgruppe substituiert ist, wobei die vorstehend erwähnten Cycloalkylteile zusätzlich durch eine Methyl- oder Ethylgruppe substituiert sein können, bedeutet,

wobei, soweit nichts anderes erwähnt wurde, unter den bei der Definition der vorstehend erwähnten Reste erwähnten Arylteilen eine Phenylgruppe zu verstehen ist, die durch $R_6$ mono- oder disubstituiert sein kann, wobei die Substituenten gleich oder verschieden sein können und

$R_6$ ein Fluor-, Chlor-, Brom- oder Jodatom, eine $C_{1-2}$-Alkyl-, Trifluormethyl- oder $C_{1-2}$-Alkoxygruppe darstellt, oder

zwei Reste $R_6$, sofern sie an benachbarte Kohlenstoffatome gebunden sind, zusammen eine $C_{3-4}$-Alkylen-, Methylendioxy-oder 1,3-Butadien-1,4-ylengruppe darstellen.

Bevorzugte Verbindungen der obigen allgemeinen Formel I sind diejenigen, in denen

$R_a$ ein Wasserstoffatom,

$R_b$ eine Benzyl- oder 1-Phenylethylgruppe oder eine durch die Reste $R_1$ und $R_2$ substituierte Phenylgruppe, wobei

$R_1$ ein Wasserstoff-, Fluor-, Chlor- oder Bromatom, eine Methyl-, Trifluormethyl-, Cyan- oder Ethinylgruppe und $R_2$ ein Wasserstoff- oder Fluoratom darstellen,

$R_c$ ein Wasserstoffatom,

X ein Stickstoffatom,

A eine 1,2-Vinylengruppe,

B eine $C_{1-4}$-Alkylengruppe,

C eine durch den Rest $R_5$ oder durch den Rest $R_5$ und eine $C_{1-4}$-Alkylgruppe substituierte 2-Oxo-morpholin-4-yl-Gruppe, wobei

$R_5$ eine $C_{3-4}$-Alkyl-, $C_{1-2}$-Alkoxy-$C_{1-4}$-alkyl-, Di-($C_{1-2}$-Alkyl)-amino-$C_{1-4}$-alkyl-, Pyrrolidino-$C_{1-4}$-alkyl-, Piperidino-$C_{1-4}$-alkyl-, Morpholino-$C_{1-4}$-alkyl-, 4-($C_{1-2}$-Alkyl)-piperazino-$C_{1-4}$-alkyl-, $C_{1-2}$-Alkylsulfanyl-$C_{1-4}$-alkyl-, $C_{1-2}$-Alkylsulfinyl-$C_{1-4}$-alkyl-, $C_{1-2}$-Alkylsulfonyl-$C_{1-4}$-alkyl-, Cyan-$C_{1-4}$-alkyl-, $C_{1-2}$-Alkoxycarbonyl-$C_{1-4}$-alkyl-, Aminocarbonyl-$C_{1-4}$-alkyl-, $C_{1-2}$-Alkyl-aminocarbonyl-$C_{1-4}$-alkyl-, Di- ($C_{1-2}$-alkyl)-aminocarbonyl-$C_{1-4}$-alkyl-, Pyrrolidinocarbonyl-$C_{1-4}$-alkyl-, Piperidinocarbonyl-$C_{1-4}$-alkyl-, Morpholinocarbonyl-$C_{1-4}$-alkyl- oder eine 4-($C_{1-2}$-Alkyl)-piperazinocarbonyl-$C_{1-4}$-alkylgruppe darstellt,

eine durch zwei Reste $R_5$ substituierte 2-Oxo-morpholin-4-yl-Gruppe, wobei $R_5$ wie vorstehend erwähnt definiert ist und die beiden Reste $R_5$ gleich oder verschieden sein können,

eine 2-Oxo-morpholin-4-yl-Gruppe, in der die beiden Wasserstoffatome einer Methylengruppe durch eine $-(CH_2)_m-$, $-CH_2-Y-CHi-$, $-CH_2-Y-CH_2-CH_2-$ oder $-CH_2CH_2-Y-CH_2CH_2-$Brücke ersetzt sind, wobei

m die Zahl 2, 3, 4 oder 5 und
Y ein Sauerstoff- oder Schwefelatom, eine Sulfinyl-, Sulfonyl- oder $C_{1-2}$-Alkylimino-Gruppe darstellen,

eine 2-Oxo-morpholin-4-yl-Gruppe, in der ein Wasserstoffatom in 5-Stellung zusammen mit einem Wasserstoffatom in 6-Stellung durch eine $-(CH_2)_n-$, $-CH_2-Y-CH_2-$, $-CH_2-Y-CH_2CH_2-$ oder $-CH_2CH_2-Y-CH_2$-Brücke ersetzt ist, wobei

Y wie vorstehend erwähnt definiert ist und
n die Zahl 2, 3 oder 4 darstellt,

oder, falls D zusammen mit E eine Gruppe $R_d$ darstellt, auch eine 2-Oxo-morpholin-4-yl-Gruppe, die durch 1 oder 2 Methyl-oder Ethylgruppen substituiert sein kann,

D eine $-O-C_{1-4}$-Alkylengruppe, wobei der Alkylenteil mit dem Rest E verknüpft ist, und

E eine Dimethylamino-, Diethylamino-, Pyrrolidino-, Piperidino-, Morpholino-, 4-Methyl-piperazino- oder 4-Ethylpiperazinogruppe oder

D zusammen mit E ein Wasserstoffatom,

eine Methoxy-, Ethoxy-, 2-Methoxy-ethoxy-, 3-Methoxy-propyloxy-, Tetrahydrofuran-3-yloxy-, Tetrahydropyran-3-yloxy-, Tetrahydropyran-4-yloxy-, Tetrahydrofuranylmethoxy- oder Tetrahydropyranylmethoxygruppe,

eine Cyclobutyloxy-, Cyclopentyloxy-, Cyclohexyloxy-, Cyclopropylmethoxy-, Cyclobutylmethoxy-, Cyclopentylmethoxy- oder Cyclohexylmethoxygruppe oder

einen Rest $R_d$ darstellen, wobei

$R_d$ eine 2-(Cyclobutyloxy)-ethoxy-, 2-(Cyclopentyloxy)-ethoxy-, 2-(Cyclopropylmethoxy)-ethoxy- oder 2-(Cyclobutylmethoxy)-ethoxygruppe darstellt,

deren Tautomere, Stereoisomere und deren Salze.

Besonders bevorzugte Verbindungen der obigen allgemeinen Formel I sind diejenigen, in denen

$R_a$ ein Wasserstoffatom,

$R_b$ eine 1-Phenylethyl-, 3-Methylphenyl-, 3-Chlorphenyl-, 3-Bromphenyl- oder 3-Chlor-4-fluorphenylgruppe,

$R_c$ ein Wasserstoffatom,

X ein Stickstoffatom,

A eine 1,2-Vinylengruppe,

B eine Methylengruppe,

C eine 2-Oxo-morpholin-4-yl-Gruppe, die durch eine Methoxymethyl-, Methoxyethyl-, Ethoxymethyl-, Ethoxyethyl-, Dimethylaminomethyl-, Dimethylaminoethyl-, Diethylaminomethyl-, Diethylaminoethyl-, Cyanmethyl- oder Cyanethylgruppe substituiert ist,

eine 2-Oxo-morpholin-4-yl-Gruppe, in der die beiden Wasserstoffatome einer Methylengruppe durch eine $-CH_2CH_2-$, $-CH_2CH_2CH_2-$, $-CH_2CH_2CH_2CH_2-$, $-CH_2CH_2CH_2CH_2CH_2-$, $-CH_2-O-CH_2CH_2-$, $-CH_2-NCH_3-CH_2CH_2-$, $-CH_2-NC_2H_5-CH_2CH_2-$, $-CH_2CH_2-O-CH_2CH_2-$, $-CH_2CH_2-NCH_3-CH_2CH_2-$ oder $-CH_2CH_2-NC_2H_5-CH_2CH_2$-Brücke ersetzt sind,

eine 2-Oxo-morpholin-4-yl-Gruppe, in der ein Wasserstoffatom in 5-Stellung zusammen mit einem Wasserstoffatom in 6-Stellung durch eine $-CH_2CH_2CH_2-$, $-CH_2CH_2CH_2CH_2-$, $-CH_2-O-CH_2-$, $-CH_2-NCH_3-CH_2-$,

$-CH_2-NC_2H_5-CH_2$, $-CH_2-O-CH_2CH_2-$, $-CH_2-NCH_3-CH_2CH_2-$, $-CH_2-NC_2H_5-CH_2CH_2-$, $-CH_2CH_2-O-CH_2-$, $-CH_2CH_2-NCH_3-CH_2-$ oder $-CH_2CH_2-NC_2H_5-CH_2$-Brücke ersetzt ist,

oder, falls D zusammen mit E eine Gruppe $R_d$ darstellt, auch eine 2-Oxo-morpholin-4-yl-Gruppe, die durch 1 oder 2 Methylgruppen substituiert ist, und

D zusammen mit E ein Wasserstoffatom,

eine Methoxy-, Ethoxy-, 2-Methoxy-ethoxy-, 3-Methoxy-propyloxy-, Tetrahydrofuran-3-yloxy-, Tetrahydropyran-4-yloxy- oder Tetrahydrofuranylmethoxygruppe,

eine Cyclobutyloxy-, Cyclopentyloxy-, Cyclopropylmethoxy-, Cyclobutylmethoxy- oder Cyclopentylmethoxygruppe oder

einen Rest $R_d$ darstellen, wobei

$R_d$ eine 2-(Cyclobutyloxy)-ethoxy-, 2-(Cyclopentyloxy)-ethoxy-, 2-(Cyclopropylmethoxy)-ethoxy- oder 2-(Cyclobutylmethoxy)-ethoxygruppe darstellt,

deren Tautomere, Stereoisomere und deren Salze.

Ganz besonders bevorzugte Verbindungen der obigen allgemeinen Formel I sind diejenigen, in denen

$R_a$ ein Wasserstoffatom,

$R_b$ eine 3-Chlor-4-fluorphenylgruppe,

$R_c$ ein Wasserstoffatom,

X ein Stickstoffatom,

A eine 1,2-Vinylengruppe,

B eine Methylengruppe,

C eine 2-Oxo-morpholin-4-yl-Gruppe, die durch eine Methoxymethyl- oder Methoxyethylgruppe substituiert ist, oder

eine 2-Oxo-morpholin-4-yl-Gruppe, in der die beiden Wasserstoffatome einer Methylengruppe durch eine $-CH_2CH_2-O-CH_2CH_2$-Brücke ersetzt sind, und

D zusammen mit E ein Wasserstoffatom, eine Methoxy- oder Cyclopropylmethoxygruppe bedeuten,

deren Tautomere, Stereoisomere und deren Salze.

Beispielsweise seien folgende besonders bevorzugte Verbindungen der obigen allgemeinen Formel I erwähnt:

(1) 4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin,

(2) 4-[(3-Chlor-4-fluor-phenyl)amino]-6-{[4-(2-oxo-1,9-dioxa-4-aza-spiro[5.5]undec-4-yl)-1-oxo-2-buten-1-yl]amino}-7-cyclopropylmethoxy-chinazolin und

(3) 4-[(3-Chlor-4-fluor-phenyl)amino]-6-({4-[2-(2-methoxyethyl)-6-oxo-morpholin-4-yl]-1-oxo-2-buten-1-yl}amino)-7-cyclopropylmethoxy-chinazolin,

deren Tautomere, Stereoisomere und deren Salze.

Die Verbindungen der allgemeinen Formel I lassen sich beispielsweise nach folgenden Verfahren herstellen:

a) Umsetzung einer gegebenenfalls im Reaktionsgemisch gebildeten Verbindung der allgemeinen Formel

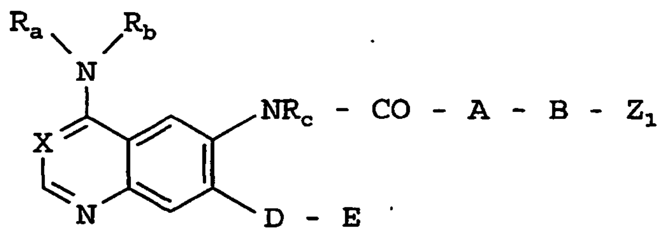

, (II)

in der

$R_a$ bis $R_c$, A, B, D, E und X wie eingangs erwähnt definiert sind und
$Z_1$ eine Austrittsgruppe wie ein Halogenatom, z.B. ein Chlor-oder Bromatom, oder eine Hydroxygruppe darstellt,
mit einer Verbindung der allgemeinen Formel

H - C , (III)

in der
C wie eingangs erwähnt definiert ist.

Die Umsetzung wird gegebenenfalls in einem Lösungsmittel oder Lösungsmittelgemisch wie Acetonitril, Methylenchlorid, Dimethylformamid, Benzol, Toluol, Chlorbenzol, Tetrahydrofuran, Benzol/Tetrahydrofuran oder Dioxan gegebenenfalls in Gegenwart einer anorganischen oder organischen Base und gegebenenfalls in Gegenwart eines aktivierenden Mittels zweckmäßigerweise bei Temperaturen zwischen -50 und 150°C, vorzugsweise bei Temperaturen zwischen -20 und 100°C, durchgeführt.

Mit einer Verbindung der allgemeinen Formel II, in der $Z_1$ eine Austrittsgruppe darstellt, wird die Umsetzung gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan zeckmäßigerweise in Gegenwart einer tertiären organischen Base wie Triethylamin oder N-Ethyl-diisopropylamin, wobei diese organischen Basen gleichzeitig auch als Lösungsmittel dienen können, oder in Gegenwart einer anorganischen Base wie Natriumkarbonat oder Kaliumcarbonat zweckmäßigerweise bei Temperaturen zwischen -50 und 150°C, vorzugsweise bei Temperaturen zwischen -20 und 100°C, durchgeführt.

Mit einer Verbindung der allgemeinen Formel II, in der $Z_1$ eine Hydroxygruppe darstellt, wird die Umsetzung vorzugsweise in Gegenwart eines aktivierenden Mittels, z.B. in Gegenwart von Thionylchlorid oder Phosphortrichlorid zweckmäßigerweise in einem Lösungsmittel wie Acetonitril, Methylenchlorid, Tetrahydrofuran, Dioxan, Toluol, Chlorbenzol, Ethylenglycoldiethylether oder Sulfolan und gegebenenfalls in Gegenwart eines Reaktionsbeschleunigers wie Natriumiodid bei Temperaturen zwischen -50 und 150°C, vorzugsweise jedoch bei Temperaturen zwischen -20 und 100°C, durchgeführt.

b) Cyclisierung einer gegebenenfalls im Reaktionsgemisch gebildeten Verbindung der allgemeinen Formel

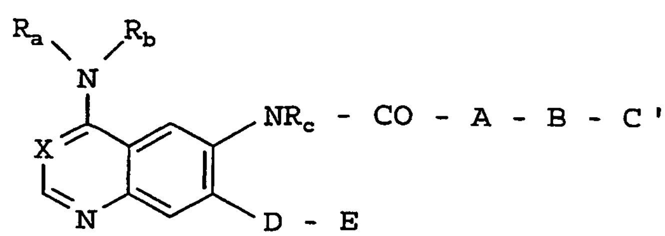

, (IV)

in der

$R_a$ bis $R_c$, A, B, D, E und X wie eingangs erwähnt definiert sind und
C' eine durch Cyclisierung in eine Gruppe C überführbare, entprechend substituierte N-(Carboxymethyl)-N-(2-hydroxyethyl)-amino- oder N-($C_{1-4}$-Alkyloxycarbonymethyl)-N-(2-hydroxyethyl)-aminogruppe bedeutet.

Die Umsetzung wird gegebenenfalls in einem Lösungsmittel oder Lösungsmittelgemisch wie Methylenchlorid, Acetonitril, Dimethylformamid, Dimethylsulfoxid, Sulfolan, Benzol, Toluol, Chlorenzol, Tetrahydrofuran, Benzol/Tetrahydrofuran oder Dioxan zweckmäßigerweise in Gegenwart einer wasserfreien Säure wie Trifluoressigsäure, Methansulfonsäure oder Schwefelsäure oder in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/-N-Hydroxysuccinimid oder 1-Hydroxy-benztriazol, N,N'-Carbonyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt.

Bei den vorstehend beschriebenen Umsetzungen können gegebenenfalls vorhandene reaktive Gruppen wie Hydroxy-, Carboxy- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Trityl-, Benzyl- oder Tetrahydropyranylgruppe,

als Schutzreste für eine Carboxygruppe die Trimethylsilyl-, Methyl-, Ethyl-, tert.Butyl-, Benzyl- oder Tetrahydropyranylgruppe und
als Schutzreste für eine Iminogruppe die Formyl-, Acetyl-, Trifluoracetyl-, Ethoxycarbonyl-, tert.Butoxycarbonyl-, Benzyloxycarbonyl-, Benzyl-, Methoxybenzyl- oder 2,4-Diethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder

Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis IV sind teilweise literaturbekannt oder man erhält diese nach an sich literaturbekannten Verfahren (siehe Beispiele I bis VII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3-(IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-$P_1$, deren Herstellung von Dexter, T. M. et al. in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:

F/L-HERc Zellen wurden in RPMI/1640 Medium (BioWhittaker), supplementiert mit 10 % foetalem Rinderserum (FCS, Boehringer Mannheim), 2 mM Glutamin (BioWhittaker), Standardantibiotika und 20 ng/ml humanem EGF (Promega), bei 37°C und 5% $CO_2$ kultiviert. Zur Untersuchung der inhibitorischen Aktivität der erfindungsgemäßen Verbindungen wurden 1,5 x $10^4$ Zellen pro Vertiefung in Triplikaten in 96-Loch-Platten in obigem Medium (200 µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1% betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96™ $AQ_{ueous}$ Non-Radioactive Cell Proliferation Assay (Promega) in O.D. Einheiten gemessen. Die relative Zellzahl wurde in Prozent der Kontrolle (F/LHERc Zellen ohne Inhibitor) berechnet und die Wirkstoffkonzentration, die die Proliferation der Zellen zu 50% hemmt ($IC_{50}$), abgeleitet. Hierbei wurden folgende Ergebnisse erhalten:

| Verbindung (Beispiel Nr.) | Hemmung der EGF-abhängigen Proliferation $IC_{50}$ [nM] |
|---|---|
| 1 | 2 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nichtallergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome,
desweiteren zur Behandlung von Nasenpolypen sowie von Polypen des Gastrointestinaltraktes unterschiedlicher Genese wie z.B. villöse oder adenomatöse Polypen des Dickdarms, aber auch von Polypen bei familiärer Polyposis coli, bei Darmpolypen im Rahmen des Gardner-Syndroms, bei Polypen im gesamten Magen-Darm-Trakt bei Peutz-Jeghers-Syndrom, bei entzündlichen Pseudopolypen, bei juvenilen Polypen, bei Colitis cystica profunda und bei Pneumatosis cystoides intestinales.

Außerdem können die Verbindungen der allgemeinen Formel I und deren physiologisch verträglichen Salze zur Behandlung von Nierenerkrankungen, insbesondere bei zystischen Veränderungen wie bei Zystennieren, zur Behandlung von Nierenzysten, die idiopathischer Genese sein können oder im Rahmen von Syndromen auftreten wie z. B. bei der tuberöser Sklerose, bei dem von-Hippel-Lindau-Syndrom, bei der Nephronophthisis und Markschwammniere sowie anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch, broncholytisch und/oder entzündungshemmend wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden oder entzündungshemmenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intrarektal, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/-Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragées, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

Herstellung der Ausgangsprodukte:

Beispiel I

4-[(3-Chlor-4-fluor-phenyl)amino]-6-[(4-{N-[(ethoxycarbonyl)-methyl]-N-((*R*)-2-hydroxy-3-methoxy-propyl)-amino}-1-oxo-2-buten-1-yl)amino]-7-cyclopropylmethoxy-chinazolin

Zu 1.29 g Bromcrotonsäure in 30 ml Methylenchlorid werden 1.34 ml Oxalylchlorid pipettiert, anschließend werden noch 65 μl N,N-Dimethylformamid zugegeben. Das Reaktionsgemisch wird ca. 45 Minuten bei Raumtemperatur gerührt, bis die Gasentwicklung beendet ist, und anschließend zur Trockne eingeengt. Das rohe Bromcrotonsäurechlorid wird in 15 ml Methylenchlorid aufgenommen und unter Eisbad-Kühlung innerhalb von fünf Minuten zu einer Lösung aus 2.00 g 6-Amino-4-[(3-chlor-4-fluor-phenyl)amino]-7-cyclopropylmethoxy-chinazolin und 2.91 ml Diisopropylethylamin in 60 ml Tetrahydrofuran getropft. Das Reaktionsgemisch wird 45 Minuten unter Eisbad-kühlung, dann zwei Stunden bei Raumtemperatur gerührt. Anschließend wird eine Lösung von 1.80 g ((*R*)-2-Hydroxy-3-methoxy-propylamino)-essigsäure-ethylester in 5 ml Tetrahydrofuran zugegeben. Das Reaktionsgemisch wird ca. 40 Stunden auf 60 °C erhitzt. Zur Aufarbeitung wird das Reaktionsgemisch eingeengt. Der Kolbenrückstand wird in 200 ml Essigester gelöst, mit 5%iger Zitronensäure-Lösung und gesättigter Natriumchlorid-Lösung gewaschen und auf etwa 100 ml eingeengt. Das Konzentrat wird über eine Kieselgelsäule mit Essigester/Methanol(100:0 bis 70:30) als Laufmittel chromatographiert. Man erhält die Titelverbindung, verunreinigt mit etwas bereits cyclisiertem Produkt, als bräunlichen Schaum.
Ausbeute: 1.10 g (32 % der Theorie),
$R_f$-Wert: 0.42 (Kieselgel, Methylenchlorid/Methanol = 9:1) Massenspektrum ($ESI^-$): m/z = 614, 616 $[M-H]^-$

Analog Beispiel I wird folgende Verbindung erhalten:

(1) 4-[(3-Chlor-4-fluor-phenyl)amino]-6-[(4-{N-[(4-hydroxytetrahydropyran-4-yl)methyl]-N-[(ethoxycarbonyl)methyl]-amino}-1-oxo-2-buten-1-yl)amino]-7-cyclopropylmethoxy-chinazolin $R_f$-Wert: 0.40 (Kieselgel, Methylenchlorid/Methanol = 9:1) Massenspektrum ($ESI^-$): m/z = 640, 642 $[M-H]^-$

(2) 4-[(3-Chlor-4-fluor-phenyl)amino]-6-[(4-{N-[(ethoxycarbonyl)methyl]-N-((*S*)-2-hydroxy-3-methoxy-propyl)-amino}-1-oxo-2-buten-1-yl)amino]-7-cyclopropylmethoxy-chinazolin
$R_f$-Wert: 0.30 (Kieselgel, Methylenchlorid/Methanol = 20:1) Massenspektrum ($ESI^+$): m/z = 616, 618 $[M+H]^+$

Beispiel II

6-Amino-4-[(3-chlor-4-fluor-phenyl)amino]-7-cyclopropylmethoxy-chinazolin

36.02 g 4-[(3-Chlor-4-fluor-phenyl)amino]-7-cyclopropylmethoxy-6-nitro-chinazolin werden in einem Gemisch aus 1080 ml Ethanol, 144 ml Eisessig und 360 ml Wasser suspendiert und zum Rückfluß erhitzt, wobei die Substanz in Lösung geht. Nun werden vorsichtig 20.70 g Eisenpulver portionsweise zugegeben. Nach 30 Minuten ist die Umsetzung vollständig und das Reaktionsgemisch wird zur Trockne eingeengt. Der Rückstand wird in 1200 ml Methylenchlorid/Methanol (9:1) aufgenommen und mit 33%iger Ammoniak-Lösung alkalisch gestellt. Der Eisenschlamm wird über einen Schnelllauffilter abgesaugt und mit 500 ml Methylenchlorid/Methanol (9:1) nachgewaschen. Das braune Filtrat wird über eine Kieselgelpackung filtriert, mit insgesamt 2000 ml Methylenchlorid/Methanol (9:1) nachgewaschen und eingeengt. Der Kolbenrückstand wird mit 140 ml Diethylether aufgeschlämmt, abgesaugt und an der Luft getrocknet.
Ausbeute: 29.70 g (89 % der Theorie),
Schmelzpunkt: 208°C
Massenspektrum ($ESI^+$): m/z = 359, 361 $[M+H]^+$

Beispiel III

4-[(3-Chlor-4-fluor-phenyl)amino]-7-cyclopropylmethoxy-6-nitro-chinazolin

29.36 g Cyclopropylmethanol werden in 310 ml N,N-Dimethylformamid gelöst und im Eisbad auf ca. 10°C gekühlt. Dann werden portionsweise 41.58 g Kalium-tert.butylat zugegeben, wobei die Temperatur unter 15°C bleiben sollte. Anschließend wird das Reaktionsgemisch noch 30 Minuten bei 10°C gerührt, dann werden portionsweise 31.19 g 4-[(3-Chlor-4-fluor-phenyl)-amino]-7-fluor-6-nitro-chinazolin zugegeben, wobei die Temperatur wiederum 15°C nicht überschreiten sollte. Das tiefrote Reaktionsgemisch wird noch eine Stunde bei 15 °C gerührt. Zur Aufarbeitung wird der Ansatz auf 2.5 1 Wasser gegossen und mit 2N Salzsäure neutralisiert. Der entstandene gelbliche Niederschlag wird abgesaugt, mit Wasser nachgewaschen und bei 50°C im Trockenschrank getrocknet.
Ausbeute: 36.02 g (100 % der Theorie),
Schmelzpunkt: 204°C
Massenspektrum ($ESI^+$): m/z = 389, 391 $[M+H]^+$

Beispiel IV

((R)-2-Hydroxy-3-methoxy-propylamino)-essigsäure-ethylester

Die unter Beispiel V erhaltene Rohproduktlösung von [N-Benzyl-N-((*R*)-2-hydroxy-3-methoxy-prop-1-yl)-amino]-essigsäure-ethylester in Ethanol wird mit weiteren 20 ml absolutem Ethanol versetzt und in Gegenwart von 500 mg Palladium (10%ig auf Aktivkohle) als Katalysator ca. vier Stunden bei Raumtemperatur hydriert, bis die berechnete Menge Wasserstoff aufgenommen ist. Zur Aufarbeitung wird der Katalysator abfiltriert und das Filtrat im Vakuum eingeengt, wobei ein zähes, farbloses Öl zurückbleibt.
Ausbeute: 1.90 g (88 % der Theorie),
$R_f$-Wert: 0.48 (Kieselgel, Methylenchlorid/Methanol = 9:1) Massenspektrum ($ESI^+$): m/z = 192 $[M+H]^+$
Analog Beispiel IV werden folgende Verbindungen erhalten:

(1) (2-Hydroxy-4-methoxy-butylamino)-essigsäure
(Die Hydrierung wird in einem Gemisch aus Methanol/Wasser = 10:1 durchgeführt.)
$R_f$-Wert: 0.80 (Reversed Phase DC-Fertigplatte (E. Merck), Acetonitril/Wasser/Trifluoressigsäure = 50:50:1)
Massenspektrum ($ESI^-$) : m/z = 176 $[M-H]^-$

(2) ((*S*)-2-Hydroxy-3-methoxy-propylamino)-essigsäureethylester
Massenspektrum (EI): m/z = 191 $[M]^+$

Beispiel V

[N-Benzyl-N-((*R*)-2-hydroxy-3-methoxy-propyl)-amino]-essigsäure-ethylester

Ein Gemisch aus 2.20 g N-Benzylamino-essigsäure-ethylester und 1.00 g (R) (-) -2- (Methoxymethyl) -oxiran (Fluka) in 10 ml absolutem Ethanol wird unter Argonatmosphäre übers Wochenende stehengelassen. Die erhaltene Rohproduktlösung wird ohne weitere Reinigung weiter umgesetzt.
$R_f$-Wert: 0.57 (Kieselgel, Cyclohexan/Essigester = 1:1) Massenspektrum ($ESI^+$): m/z = 282 $[M+H]^+$
Analog Beispiel V werden folgende Verbindungen erhalten:

(1) [N-Benzyl-N-(2-hydroxy-4-methoxy-butyl)-amino]-essigsäure (Die Reaktion wird mit N-Benzylglycin in 1N Natronlauge durchgeführt.)
$R_f$-Wert: 0.57 (Reversed Phase DC-Fertigplatte (E. Merck), Acetonitril/Wasser/Trifluoressigsäure = 50:50:1) Massenspektrum ($ESI^-$): m/z = 266 $[M-H]^-$

(2) [N-Benzyl-N-((*S*)-2-hydroxy-3-methoxy-propyl)-amino]-essigsäure-ethylester
$R_f$-Wert: 0.57 (Kieselgel, Cyclohexan/Essigester = 1:1) Massenspektrum ($ESI^+$): m/z = 282 $[M+H]^+$

Beispiel VI

(4-Hydroxy-tetrahydropyran-4-yl)methylamino]-essigsäure-ethylester

5.30 g Glycinethylester-hydrochlorid werden in 10 ml gesättigter Kaliumcarbonat-Lösung gelöst. Dann werden 10 g festes Kaliumcarbonat unter Eisbad-Kühlung zugegeben. Die entstandene Masse wird mehrmals gründlich mit Diethylether extrahiert. Die vereinigten Etherextrakte werden über Natriumsulfat getrocknet und eingeengt. Der so erhaltene Glycinethylester wird zusammen mit 4.20 g 1,6-Dioxa-spiro[2.5]octan in 20 ml absolutem Ethanol gelöst und in einer Roth-Bombe ca. sechs Stunden auf 90°C erhitzt. Nach Abkühlung auf Raumtemperatur wird das Reaktionsgemisch eingeengt. Das gelbliche, ölige Rohprodukt wird ohne weitere Reinigung weiter umgesetzt.
Massenspektrum ($ESI^+$) : m/z = 240 $[M+Na]^+$

Beispiel VII

(2-Hydroxy-4-methoxy-butylamino)-essigsäure-methylester-hydrochlorid

Zu einer Suspension aus 5.80 g (2-Hydroxy-4-methoxy-butylamino)-essigsäure in 200 ml Methanol werden unter Eisbad-Kühlung 11.94 ml Thionylchlorid innerhalb von 20 Minuten getropft. Man läßt das Reaktionsgemisch über Nacht auf Raumtemperatur erwärmen. Zur Aufarbeitung wird die trübe Lösung zur Trockne eingeengt. Der Rückstand

wir mehrmals mit jeweils 100 ml Methanol verrührt, welches dann am Rotationsverdampfer im Vakuum abdestilliert wird. Das zähe Rohprodukt wird ohne weitere Reinigung weiter umgesetzt.
Ausbeute: 8.70 g,
Massenspektrum ($ESI^+$) : m/z = 192 $[M+H]^+$

Herstellung der Endverbindungen:

Beispiel 1

4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin

950 mg 4-[(3-Chlor-4-fluor-phenyl)amino]-6-[(4-{N-[(ethoxycarbonyl)methyl]-N-((*R*)-2-hydroxy-3-methoxy-propyl)-amino}-1-oxo-2-buten-1-yl)amino]-7-cyclopropylmethoxy-chinazolin und 195 µl Methansulfonsäure in 10 ml Acetonitril werden etwa vier Stunden unter Rückfluß erhitzt. Zur Aufarbeitung wird das Reaktionsgemisch in einem Eiswasserbad abgekühlt, mit 75 ml Essigester und 25 ml gesättigter Natriumhydrogencarbonat-Lösung versetzt und 10 Minuten kräftig durchgerührt. Die organische Phase wird abgetrennt, mit gesättigter Natriumhydrogencarbonat-Lösung und gesättigter Natriumchlorid-Lösung gewaschen und über Magnesiumsulfat getrocknet. Das Lösungsmittel wird im Vakuum abdestilliert, wobei ein bräunlicher Schaum zurückbleibt.
Ausbeute: 610 mg (69 % der Theorie),
$R_f$-Wert: 0.55 (Kieselgel, Methylenchlorid/Methanol = 9:1) Massenspektrum ($ESI^+$) : m/z = 570, 572 $[M+H]^+$

Analog Beispiel 1 werden folgende Verbindungen erhalten:

(1) 4-[(3-Chlor-4-fluor-phenyl)amino]-6-{[4-(2-oxo-1,9-dioxa-4-aza-spiro[5.5]undec-4-yl)-1-oxo-2-buten-1-yl]amino}-7-cyclopropylmethoxy-chinazolin
$R_f$-Wert: 0.53 (Kieselgel, Methylenchlorid/Methanol = 15:1) Massenspektrum ($ESI^-$): m/z = 594, 596 $[M-H]^-$

(2) 4-[(3-Chlor-4-fluor-phenyl)amino]-6-{[4-((*S*)-2-methoxymethyl-6-oxo-morpholin-4-yl)-1-oxo-2-buten-1-yl]amino}-7-cyclopropylmethoxy-chinazolin
$R_f$-Wert: 0.53 (Kieselgel, Methylenchlorid/Methanol = 9:1) Massenspektrum ($ESI^-$): m/z = 568, 570 $[M-H]^-$

Beispiel 2

4-[(3-Chlor-4-fluor-phenyl)amino]-6-({4-[2-(2-methoxy-ethyl)-6-oxo-morpholin-4-yl]-1-oxo-2-buten-1-yl}amino)-7-cyclopropylmethoxy-chinazolin

Zu 1.61 g Bromcrotonsäure in 50 ml Methylenchlorid werden 1.68 ml Oxalylchlorid pipettiert, anschließend wird noch ein Tropfen N,N-Dimethylformamid zugegeben. Das Reaktionsgemisch wird etwa eine Stunde bei Raumtemperatur gerührt, bis die Gasentwicklung beendet ist, und anschließend zur Trockne eingeengt. Das rohe Bromcrotonsäurechlorid wird in 20 ml Methylenchlorid aufgenommen und unter Eisbad-Kühlung innerhalb von fünf Minuten zu einer Lösung aus 2.50 g 6-Amino-4-[(3-chlor-4-fluor-phenyl)amino]-7-cyclopropylmethoxy-chinazolin und 12.14 ml Diisopropylethylamin in 75 ml Tetrahydrofuran getropft. Das Reaktionsgemisch wird eine Stunde unter Eisbad-Kühlung, dann weitere zwei Stunden bei Raumtemperatur gerührt. Anschließend wird eine Lösung aus 8.20 g (2-Hydroxy-4-methoxybutylamino)-essigsäure-methylester-hydrochlorid in 15 ml N,N-Dimethylformamid in einer Portion zugegeben. Das Reaktionsgemisch wird 24 Stunden bei 75°C gerührt. Zur Aufarbeitung wird das Reaktionsgemisch zur Trockne eingeengt und der Kolbenrückstand zwischen 250 ml Essigester und 200 ml 5%iger Zitronensäure-Lösung verteilt. Die organische Phase wird mit gesättigter Natriumchlorid-Lösung gewaschen, über Magnesiumsulfat getrocknet und eingeengt. Das Rohprodukt wird chromatographisch über ein Kieselgelsäule mit Essigester als Laufmittel gereinigt. Man erhält das cyclisierte Produkt als beigefarbenen Feststoff.
Ausbeute: 825 mg (20 % der Theorie),
$R_f$-Wert: 0.38 (Kieselgel, Essigester)
Massenspektrum ($ESI^-$): m/z = 582, 584 $[M-H]^-$

Analog den vorstehenden Beispielen und anderen literaturbekannten Methoden können folgende Verbindungen hergestellt werden:

(1) 4-[(3-Chlor-4-fluor-phenyl)amino]-6-{[4-((*S*)-2-methoxymethyl-6-oxo-morpholin-4-yl)-1-oxo-2-buten-1-yl)amino}-7-methoxy-chinazolin

(2) 4-[(3-Chlor-4-fluor-phenyl)amino]-6-{[4-((*S*)-2-methoxymethyl-6-oxo-morpholin-4-yl)-1-oxo-2-buten-1-yl]amino}-chinazolin

(3) 4-[(*R*)-(1-Phenyl-ethyl)amino]-6-({4-[2-(2-methoxy-ethyl)-6-oxo-morpholin-4-yl]-1-oxo-2-buten-1-yl}amino)-chinazolin

(4) 4-[(3-Chlor-4-fluor-phenyl)amino]-6-({4-[3-(2-methoxyethyl)-2-oxo-morpholin-4-yl]-1-oxo-2-buten-1-yl}amino)-7-cyclopropylmethoxy-chinazolin

(5) 4-[(*R*)-(1-Phenyl-ethyl)amino]-6-{[4-(2-oxo-1,9-dioxa-4-aza-spiro[5.5]undec-4-yl)-1-oxo-2-buten-1-yl]amino}-7-methoxy-chinazolin

(6) 4-[(3-Chlor-4-fluor-phenyl)amino]-6-{[4-(2-oxo-perhydrocyclopenta[1,4]oxazin-4-yl)-1-oxo-2-buten-1-yl]amino}-7-methoxy-chinazolin

(7) 4-[(3-Chlor-4-fluor-phenyl)amino]-6-{[4-(5-oxo-perhydro-2,4-dioxa-7-aza-inden-7-yl)-1-oxo-2-buten-1-yl]amino}-7-methoxy-chinazolin

Beispiel 3

Dragées mit 75 mg Wirksubstanz

| | | |
|---|---|---|
| 1 | Dragéekern enthält: | |
| | Wirksubstanz | 75,0 mg |
| | Calciumphosphat | 93,0 mg |
| | Maisstärke | 35,5 mg |
| | Polyvinylpyrrolidon | 10,0 mg |
| | Hydroxypropylmethylcellulose | 15,0 mg |
| | Magnesiumstearat | 1,5 mg |
| | | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht | 230 mg |
| Stempel | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht | 245 mg. |

Beispiel 4

Tabletten mit 100 mg Wirksubstanz

Zusammensetzung:

| | | |
|---|---|---|
| 1 | Tablette enthält: | |

(fortgesetzt)

| | | |
|---|---|---|
| | Wirksubstanz | 100,0 mg |
| | Milchzucker | 80,0 mg |
| | Maisstärke | 34,0 mg |
| | Polyvinylpyrrolidon | 4,0 mg |
| | Magnesiumstearat | 2,0 mg |
| | | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht | 220 mg |
| Durchmesser | 10 mm, |

biplan mit beidseitiger Facette und einseitiger Teilkerbe.

Beispiel 5

Tabletten mit 150 mg Wirksubstanz

Zusammensetzung:

| | | |
|---|---|---|
| 1 | Tablette enthält: | |
| | Wirksubstanz | 150,0 mg |
| | Milchzucker pulv. | 89,0 mg |
| | Maisstärke | 40,0 mg |
| | Kolloide Kieselgelsäure | 10,0 mg |
| | Polyvinylpyrrolidon | 10,0 mg |
| | Magnesiumstearat | 1,0 mg |
| | | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht | 300 mg |
| Stempel | 10 mm, flach |

Beispiel 6

Hartgelatine-Kapseln mit 150 mg Wirksubstanz

| | | |
|---|---|---|
| 1 | Kapsel enthält: | |
| | Wirkstoff | 150,0 mg |
| | Maisstärke getr. ca. | 180,0 mg |

(fortgesetzt)

| | | |
|---|---|---|
| | Milchzucker pulv. ca. | 87,0 mg |
| | Magnesiumstearat | 3,0 mg |
| | ca. | 420,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung | ca. 320 mg |
| Kapselhülle | Hartgelatine-Kapsel Größe 1. |

Beispiel 7

Suppositorien mit 150 mg Wirksubstanz

| | | |
|---|---|---|
| 1 | Zäpfchen enthält: | |
| | Wirkstoff | 150,0 mg |
| | Polyäthylenglykol 1500 | 550,0 mg |
| | Polyäthylenglykol 6000 | 460,0 mg |
| | Polyoxyäthylensorbitanmonostearat | 840,0 mg |
| | | 2 000,0 mg |

Herstellung:

Nach dem Aufschmelzen der Suppositorienmasse wird der Wirkstoff darin homogen verteilt und die Schmelze in vorgekühlte Formen gegossen.

Beispiel 8

Suspension mit 50 mg Wirksubstanz

| | |
|---|---|
| 100 ml Suspension enthalten: | |
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

Beispiel 9

Ampullen mit 10 mg Wirksubstanz

Zusammensetzung:

| | | |
|---|---|---|
| Wirkstoff | | 10,0 mg |
| 0.01N Salzsäure s.q. | | |
| Aqua bidest | ad | 2,0 ml |

Herstellung:

Die Wirksubstanz wird in der erforderlichen Menge 0,01N HCl gelöst, mit Kochsalz isotonisch gestellt, sterilfiltriert und in 2 ml Ampullen abgefüllt.

Beispiel 10

Ampullen mit 50 mg Wirksubstanz

Zusammensetzung:

| | | |
|---|---|---|
| Wirkstoff | | 50,0 mg |
| 0,01N Salzsäure s.q. | | |
| Aqua bidest | ad | 10,0 ml |

Herstellung:

Die Wirksubstanz wird in der erforderlichen Menge 0,01N HCl gelöst, mit Kochsalz isotonisch gestellt, sterilfiltriert und in 10 ml Ampullen abgefüllt.

Beispiel 11

Kapseln zur Pulverinhalation mit 5 mg Wirksubstanz

| | | |
|---|---|---|
| 1 | Kapsel enthält: | |
| | Wirksubstanz | 5,0 mg |
| | Lactose für Inhalationszwecke | 15,0 mg |
| | | 20,0 mg |

Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.

| | |
|---|---|
| Kapselgewicht | 70,0 mg |
| Kapselgröße | 3 |

Beispiel 12

Inhalationslösung für Handvernebler mit 2,5 mg Wirksubstanz

| | | | |
|---|---|---|---|
| 1 | Hub enthält: | | |
| | Wirksubstanz | | 2,500 mg |
| | Benzalkoniumchlorid | | 0,001 mg |
| | 1N-Salzsäure q.s. | | |
| | Ethanol/Wasser (50/50) | ad | 15,000 mg |

Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.

| | |
|---|---|
| Füllmasse des Behälters | 4,5 g |

**Patentansprüche**

**1.** Bicyclische Heterocyclen der allgemeinen Formel

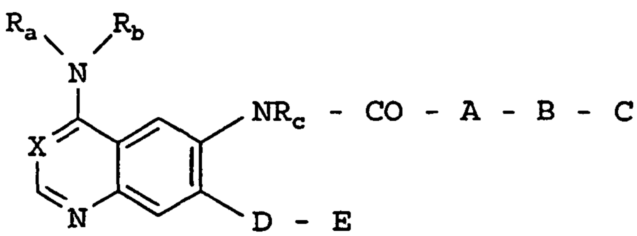

, (I)

in der

$R_a$ ein Wasserstoffatom oder eine Methylgruppe,

$R_b$ eine Phenyl-, Benzyl- oder 1-Phenylethylgruppe, in denen der Phenylkern jeweils durch die Reste $R_1$ bis $R_3$ substituiert ist, wobei

$R_1$ und $R_2$, die gleich oder verschieden sein können, jeweils ein Wasserstoff-, Fluor-, Chlor-, Brom- oder Jodatom,

eine Methyl-, Ethyl-, Hydroxy-, Methoxy-, Ethoxy-, Amino-, Cyan-, Vinyl- oder Ethinylgruppe,

eine Aryl-, Aryloxy-, Arylmethyl- oder Arylmethoxygruppe,

eine durch 1 bis 3 Fluoratome substituierte Methyl- oder Methoxygruppe oder

$R_1$ zusammen mit $R_2$, sofern diese an benachbarte Kohlenstoffatome gebunden sind, eine -CH=CH-CH=CH-, -CH=CH-NH-oder -CH=N-NH-Gruppe und

$R_3$ ein Wasserstoff-, Fluor-, Chlor- oder Bromatom,

$R_c$ ein Wasserstoffatom oder eine Methylgruppe,

X eine durch eine Cyangruppe substituierte Methingruppe oder ein Stickstoffatom,

A eine 1,1- oder 1,2-Vinylengruppe, die jeweils durch eine oder zwei Methylgruppen oder durch eine Trifluormethylgruppe substituiert sein kann,

eine Ethinylengruppe oder

eine gegebenenfalls durch eine Methyl- oder Trifluormethylgruppe substituierte 1,3-Butadien-1,4-ylengruppe,

B eine Alkylen- oder -CO-alkylen-gruppe, in denen der Alkylenteil jeweils 1 bis 4 Kohlenstoffatome enthält, wobei die Verknüpfung der -CO-alkylengruppe mit der benachbarten Gruppe A jeweils über die Carbonylgruppe erfolgen muß,

eine -CO-O-alkylen- oder -CO-$NR_4$-alkylen-Gruppe, in denen der Alkylenteil jeweils 1 bis 4 Kohlenstoffatome enthält, wobei die Verknüpfung mit der benachbarten Gruppe A jeweils über die Carbonylgruppe erfolgen muß, in der

> $R_4$ ein Wasserstoffatom oder eine Methyl- oder Ethylgruppe darstellt,
> oder eine Carbonylgruppe,

C eine durch den Rest $R_5$ oder durch den Rest $R_5$ und eine $C_{1-4}$-Alkylgruppe substituierte 2-Oxo-morpholin-4-yl-Gruppe, wobei

> $R_5$ eine $C_{1-4}$-Alkyl-, Hydroxy-$C_{1-4}$-alkyl-, $C_{1-4}$-Alkoxy-$C_{1-4}$-alkyl-, Di- ($C_{1-4}$-Alkyl) -amino-$C_{1-4}$-alkyl-, Pyrrolidino-$C_{1-4}$-alkyl-, Piperidino-$C_{1-4}$-alkyl-, Morpholino-$C_{1-4}$-alkyl-, 4- ($C_{1-4}$-Alkyl) -piperazino-$C_{1-4}$-alkyl-, $C_{1-4}$-Alkylsulfanyl-$C_{1-4}$-alkyl-, $C_{1-4}$-Alkylsulfinyl-$C_{1-4}$-alkyl-, $C_{1-4}$-Alkylsulfonyl-$C_{1-4}$-alkyl-, Cyan-$C_{1-4}$-alkyl-, $C_{1-4}$-Alkoxycarbonyl-$C_{1-4}$-alkyl-, Aminocarbonyl-$C_{1-4}$-alkyl-, $C_{1-4}$-Alkyl-aminocarbonyl-$C_{1-4}$-alkyl-, Di-($C_{1-4}$-alkyl)aminocarbonyl-$C_{1-4}$-alkyl-, Pyrrolidinocarbonyl-$C_{1-4}$-alkyl-, Piperidinocarbonyl-$C_{1-4}$-alkyl-, Morpholinocarbonyl-$C_{1-4}$-alkyl-, oder eine 4-($C_{1-4}$-Alkyl)-piperazinocarbonyl-$C_{1-4}$-alkylgruppe darstellt,

eine durch zwei Reste $R_5$ substituierte 2-Oxo-morpholin-4-yl-Gruppe, wobei $R_5$ wie vorstehend erwähnt definiert ist und.die beiden Reste $R_5$ gleich oder verschieden sein können,

eine 2-Oxo-morpholin-4-yl-Gruppe, in der die beiden Wasserstoffatome einer Methylengruppe durch eine gegebenenfalls durch eine oder zwei $C_{1-2}$-Alkylgruppen substituierte $-(CH_2)_m-$, $-CH_2-Y-CH_2-$, $-CH_2-Y-CH_2-CH_2-$, $-CH_2CH_2-Y-CH_2CH_2-$ oder $-CH_2CH_2-Y-CH_2CH_2CH_2-$Brücke ersetzt sind, wobei

> m die Zahl 2, 3, 4, 5 oder 6 und
> Y ein Sauerstoff- oder Schwefelatom, eine Sulfinyl-, Sulfonyl- oder $C_{1-4}$-Alkylimino-Gruppe darstellen,

eine 2-Oxo-morpholin-4-yl-Gruppe, in der ein Wasserstoffatom in 5-Stellung zusammen mit einem Wasserstoffatom in 6-Stellung durch eine $-(CH_2)_n-$, $-CH_2-Y-CH_2-$, $-CH_2-Y-CH_2CH_2-$ oder $-CH_2CH_2-Y-CH_2-$Brücke ersetzt ist, wobei

> Y wie vorstehend erwähnt definiert ist und
> n die Zahl 2, 3 oder 4 darstellt,

oder, falls D zusammen mit E eine Gruppe $R_d$ darstellt, auch eine 2-Oxo-morpholin-4-yl-Gruppe, die durch 1 bis 4 $C_{1-2}$-Alkylgruppen substituiert sein kann,

D eine -O-$C_{1-6}$-Alkylengruppe, wobei der Alkylenteil mit dem Rest E verknüpft ist, oder

ein Sauerstoffatom, wobei dieses nicht mit einem Stickstoffatom des Restes E verknüpft sein kann, und

E eine durch 2 $C_{1-4}$-Alkylgruppen substituierte Aminogruppe, in der die Alkylreste gleich oder verschieden sein können und jeder Alkylteil ab Position 2 durch eine $C_{1-4}$-Alkoxy-, oder Di-($C_{1-4}$-Alkyl)-aminogruppe oder durch eine 4- bis 7-gliedrige Alkyleniminogruppe substituiert sein kann, wobei in den vorstehend erwähnten 6- bis 7-gliedrigen Alkyleniminogruppen jeweils eine Methylengruppe in 4-Stellung durch ein Sauerstoff- oder

Schwefelatom, durch eine Sulfinyl-, Sulfonyl- oder N-($C_{1-4}$-Alkyl)-iminogruppe ersetzt sein kann,

eine gegebenenfalls durch 1 bis 4 Methylgruppen substituierte 4- bis 7-gliedrige Alkyleniminogruppe,

eine gegebenenfalls durch 1 oder 2 Methylgruppen substituierte 6- bis 7-gliedrige Alkyleniminogruppe, in der jeweils eine Methylengruppe in 4-Stellung durch ein Sauerstoff- oder Schwefelatom, durch eine Sulfinyl-, Sulfonyl- oder N-($C_{1-4}$-Alkyl)-iminogruppe ersetzt ist,

eine gegebenenfalls durch 1 bis 3 Methylgruppen substituierte Imidazolylgruppe,

eine $C_{5-7}$-Cycloalkylgruppe, in der eine Methylengruppe durch ein Sauerstoff- oder Schwefelatom, durch eine Sulfinyl-, Sulfonyl-oder N-($C_{1-4}$-Alkyl)-iminogruppe ersetzt ist, oder

D zusammen mit E ein Wasserstoffatom,

eine gegebenenfalls ab Position 2 durch eine Hydroxy- oder $C_{1-4}$-Alkoxygruppe substituierte $C_{1-6}$-Alkoxygruppe,

eine $C_{3-7}$-Cycloalkoxy- oder $C_{3-7}$-Cycloalkyl-$C_{1-4}$-alkoxygruppe,

oder einen Rest $R_d$ darstellen, wobei

$R_d$ eine $C_{2-6}$-Alkoxygruppe, die ab Position 2 durch eine $C_{4-7}$-Cycloalkoxy- oder $C_{3-7}$-Cycloalkyl-$C_{1-3}$-alkoxygruppe substituiert ist,

eine $C_{4-7}$-Cycloalkoxy- oder $C_{3-7}$-Cycloalkyl-$C_{1-6}$-alkoxygruppe, in denen der Cycloalkylteil jeweils durch eine $C_{1-4}$-Alkyl-, $C_{1-4}$-Alkoxy-, Di- ($C_{1-4}$-alkyl) -amino-, Pyrrolidino-, Piperidino-, Morpholino-, Piperazino-, 4-($C_{1-2}$-Alkyl)-piperazino-, $C_{1-4}$-Alkoxy-$C_{1-2}$-alkyl-, Di- ($C_{1-4}$-alkyl) -amino-$C_{1-2}$-alkyl-, Pyrrolidino-$C_{1-2}$-alkyl-, Piperidino-$C_{1-2}$-alkyl-, Morpholino-$C_{1-2}$-alkyl-, Piperazino-$C_{1-2}$-alkyl- oder 4-($C_{1-2}$-Alkyl)-giperazino-$C_{1-2}$-alkylgruppe substituiert ist, wobei die vorstehend erwähnten Cycloalkylteile zusätzlich durch eine Methyl- oder Ethylgruppe substituiert sein können, bedeutet,

wobei, soweit nichts anderes erwähnt wurde, unter den bei der Definition der vorstehend erwähnten Reste erwähnten Arylteilen eine Phenylgruppe zu verstehen ist, die durch $R_6$ mono- oder disubstituiert sein kann, wobei die Substituenten gleich oder verschieden sein können und

$R_6$ ein Fluor-, Chlor-, Brom- oder Jodatom, eine $C_{1-2}$-Alkyl-, Trifluormethyl- oder $C_{1-2}$-Alkoxygruppe darstellt, oder

zwei Reste $R_6$, sofern sie an benachbarte Kohlenstoffatome gebunden sind, zusammen eine $C_{3-4}$-Alkylen-, Methylendioxy-oder 1,3-Butadien-1,4-ylengruppe darstellen,

deren Tautomere, Stereoisomere und deren Salze.

**2.** Bicyclische Heterocyclen der allgemeinen Formel I gemäß Anspruch 1, in der

$R_a$ ein Wasserstoffatom,

$R_b$ eine Benzyl- oder 1-Phenylethylgruppe oder eine durch die Reste $R_1$ und $R_2$ substituierte Phenylgruppe, wobei

$R_1$ ein Wasserstoff-, Fluor-, Chlor- oder Bromatom, eine Methyl-, Trifluormethyl-, Cyan- oder Ethinylgruppe und
$R_2$ ein Wasserstoff- oder Fluoratom darstellen,

$R_c$ ein Wasserstoffatom,

X ein Stickstoffatom,

A eine 1,2-Vinylengruppe,

B eine $C_{1-4}$-Alkylengruppe,

C eine durch den Rest $R_5$ oder durch den Rest $R_5$ und eine $C_{1-4}$-Alkylgruppe substituierte 2-Oxo-morpholin-4-yl-Gruppe, wobei

$R_5$ eine $C_{3-4}$-Alkyl-, $C_{1-2}$-Alkoxy-$C_{1-4}$-alkyl-, Di-($C_{1-2}$-Alkyl)-amino-$C_{1-4}$-alkyl-, Pyrrolidino-$C_{1-4}$-alkyl-, Piperidino-$C_{1-4}$-alkyl-, Morpholino-$C_{1-9}$-alkyl-, 4-($C_{1-2}$-Alkyl)-piperazino-$C_{1-4}$-alkyl-, $C_{1-2}$-Alkylsulfanyl-$C_{1-4}$-alkyl-, $C_{1-2}$-Alkylsulfinyl-$C_{1-4}$-alkyl-, $C_{1-2}$-Alkylsulfonyl-$C_{1-4}$-alkyl-, Cyan-$C_{1-4}$-alkyl-, $C_{1-2}$-Alkoxy-carbonyl-$C_{1-4}$-alkyl-, Aminocarbonyl-$C_{1-4}$-alkyl-, $C_{1-2}$-Alkyl-aminocarbonyl-$C_{1-4}$-alkyl-, Di-($C_{1-2}$-alkyl)-aminocarbonyl-$C_{1-4}$-alkyl-, Pyrrolidinocarbonyl-$C_{1-4}$-alkyl-, Piperidinocarbonyl-$C_{1-4}$-alkyl-, Morpholinocarbonyl-$C_{1-4}$-alkyl-, oder eine 4-($C_{1-2}$-Alkyl)-piperazinocarbonyl-$C_{1-4}$-alkylgruppe darstellt,

eine durch zwei Reste $R_5$ substituierte 2-Oxo-morpholin-4-yl-Gruppe, wobei $R_5$ wie vorstehend erwähnt definiert ist und die beiden Reste $R_5$ gleich oder verschieden sein können,

eine 2-Oxo-morpholin-4-yl-Gruppe, in der die beiden Wasserstoffatome einer Methylengruppe durch eine $-(CH_2)_m-$, $-CH_2-Y-CH_2-$, $-CH_2-Y-CH_2-CH_2-$ oder $-CH_2CH_2-Y-CH_2CH_2-$Brücke ersetzt sind, wobei

m die Zahl 2, 3, 4 oder 5 und
Y ein Sauerstoff- oder Schwefelatom, eine Sulfinyl-, Sulfonyl- oder $C_{1-2}$-Alkylimino-Gruppe darstellt,

eine 2-Oxo-morpholin-4-yl-Gruppe, in der ein Wasserstoffatom in 5-Stellung zusammen mit einem Wasserstoffatom in 6-Stellung durch eine $-(CH_2)_n-$, $-CH_2-Y-CH_2-$, $-CH_2-Y-CH_2CH_2-$ oder $-CH_2CH_2-Y-CH_2-$Brücke ersetzt ist, wobei

Y wie vorstehend erwähnt definiert ist und
n die Zahl 2, 3 oder 4 darstellt,

oder, falls D zusammen mit E eine Gruppe $R_d$ darstellt, auch eine 2-Oxo-morpholin-4-yl-Gruppe, die durch 1 oder 2 Methyl-oder Ethylgruppen substituiert sein kann,

D eine -O-$C_{1-4}$-Alkylengruppe, wobei der Alkylenteil mit dem Rest E verknüpft ist, und

E eine Dimethylamino-, Diethylamino-, Pyrrolidino-, Piperidino-, Morpholino-, 4-Methyl-piperazino- oder 4-Ethyl-piperazinogruppe oder

D zusammen mit E ein Wasserstoffatom,

eine Methoxy-, Ethoxy-, 2-Methoxy-ethoxy-, 3-Methoxy-propyloxy-, Tetrahydrofuran-3-yloxy-, Tetrahydropyran-3-yloxy-, Tetrahydropyran-4-yloxy-, Tetrahydrofuranylmethoxy- oder Tetrahydropyranylmethoxygruppe,

eine Cyclobutyloxy-, Cyclopentyloxy-, Cyclohexyloxy-, Cyclopropylmethoxy-, Cyclobutylmethoxy-, Cyclopentylmethoxy- oder Cyclohexylmethoxygruppe oder

einen Rest $R_d$ darstellen, wobei

$R_d$ eine 2-(Cyclobutyloxy)-ethoxy-, 2-(Cyclopentyloxy)-ethoxy-, 2-(Cyclopropylmethoxy)-ethoxy- oder 2-(Cyclobutylmethoxy)-ethoxygruppe darstellt,

deren Tautomere, Stereoisomere und deren Salze.

**3.** Bicyclische Heterocyclen der allgemeinen Formel I gemäß Anspruch 1, in der

$R_a$ ein Wasserstoffatom,

$R_b$ eine 1-Phenylethyl-, 3-Methylphenyl-, 3-Chlorphenyl-, 3-Bromphenyl- oder 3-Chlor-4-fluorphenylgruppe,

$R_c$ ein Wasserstoffatom,

X ein Stickstoffatom,

A eine 1,2-Vinylengruppe,

B eine Methylengruppe,

C eine 2-Oxo-morpholin-4-yl-Gruppe, die durch eine Methoxymethyl-, Methoxyethyl-, Ethoxymethyl-, Ethoxyethyl-, Dimethylaminomethyl-, Dimethylaminoethyl-, Diethylaminomethyl-, Diethylaminoethyl-, Cyanmethyl- oder Cyanethylgruppe substituiert ist,

eine 2-Oxo-morpholin-4-yl-Gruppe, in der die beiden Wasserstoffatome einer Methylengruppe durch eine $-CH_2CH_2-$, $-CH_2CH_2CH_2-$, $-CH_2CH_2CH_2CH_2-$, $-CH_2CH_2CH_2CH_2CH_2-$, $-CH_2-O-CH_2CH_2-$, $-CH_2-NCH_3-CH_2CH_2-$, $-CH_2-NC_2H_5-CH_2CH_2-$, $-CH_2CH_2-O-CH_2CH_2-$, $-CH_2CH_2-NCH_3-CH_2CH_2-$ oder $-CH_2CH_2-NC_2H_5-CH_2CH_2-$Brücke ersetzt sind,

eine 2-Oxo-morpholin-4-yl-Gruppe, in der ein Wasserstoffatom in 5-Stellung zusammen mit einem Wasserstoffatom in 6-Stellung durch eine $-CH_2CH_2CH_2-$, $-CH_2CH_2CH_2CH_2-$, $-CH_2-O-CH_2-$, $-CH_2-NCH_3-CH_2-$, $-CH_2-NC_2H_5-CH_2$, $-CH_2-O-CH_2CH_2-$, $-CH_2-NCH_3-CH_2CH_2-$, $-CH_2-NC_2H_5-CH_2CH_2-$, $-CH_2CH_2-O-CH_2-$, $-CH_2CH_2-NCH_3-CH_2-$ oder $-CH_2CH_2-NC_2H_5-CH_2-$Brücke ersetzt ist,

oder, falls D zusammen mit E eine Gruppe $R_d$ darstellt, auch eine 2-Oxo-morpholin-4-yl-Gruppe, die durch 1 oder 2 Methylgruppen substituiert ist, und

D zusammen mit E ein Wasserstoffatom,

eine Methoxy-, Ethoxy-, 2-Methoxy-ethoxy-, 3-Methoxy-propyloxy-, Tetrahydrofuran-3-yloxy-, Tetrahydropyran-4-yloxy- oder Tetrahydrofuranylmethoxygruppe,

eine Cyclobutyloxy-, Cyclopentyloxy-, Cyclopropylmethoxy-, Cyclobutylmethoxy- oder Cyclopentylmethoxygruppe oder

einen Rest $R_d$ darstellen, wobei

$R_d$ eine 2-(Cyclobutyloxy)-ethoxy-, 2-(Cyclopentyloxy)-ethoxy-, 2-(Cyclopropylmethoxy)-ethoxy- oder 2-(Cyclobutylmethoxy)-ethoxygruppe darstellt, deren Tautomere, Stereoisomere und deren Salze.

**4.** Bicyclische Heterocyclen der allgemeinen Formel I gemäß Anspruch 1, in der

$R_a$ ein Wasserstoffatom,

$R_b$ eine 3-Chlor-4-fluorphenylgruppe,

$R_c$ ein Wasserstoffatom,

X ein Stickstoffatom,

A eine 1,2-Vinylengruppe,

B eine Methylengruppe,

C eine 2-Oxo-morpholin-4-yl-Gruppe, die durch eine Methoxymethyl- oder Methoxyethylgruppe substituiert ist, oder

eine 2-Oxo-morpholin-4-yl-Gruppe, in der die beiden Wasserstoffatome einer Methylengruppe durch eine $-CH_2CH_2-O-CH_2CH_2-$Brücke ersetzt sind, und

D zusammen mit E ein Wasserstoffatom, eine Methoxy- oder Cyclopropylmethoxygruppe bedeuten,

deren Tautomere, Stereoisomere und deren Salze.

**5.** Folgende Verbindungen der allgemeinen Formel I gemäß Anspruch 1:

(1) 4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin,

(2) 4-[(3-Chlor-4-fluor-phenyl)amino]-6-{[4-(2-oxo-1,9-dioxa-4-aza-spiro[5.5]undec-4-yl)-1-oxo-2-buten-1-yl]amino}-7-cyclopropylmethoxy-chinazolin und

(3) 4-[(3-Chlor-4-fluor-phenyl)amino]-6-({4-[2-(2-methoxyethyl)-6-oxo-morpholin-4-yl]-1-oxo-2-buten-1-yl}amino)-7-cyclopropylmethoxy-chinazolin,

deren Tautomere, Stereoisomere und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Behandlung von benignen oder malignen Tumoren, zur Vorbeugung und Behandlung von Erkrankungen der Atemwege und der Lunge; zur Behandlung von Polypen, von Erkrankungen des Magen-Darm-Traktes, der Gallengänge und -blase sowie der Niere und der Haut geeignet ist.

**9.** Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der Verbindungen der allgemeinen Formel I gemäß den Ansprüchen 1 bis 6, **dadurch gekennzeichnet, daß**

a) eine gegebenenfalls im Reaktionsgemisch gebildete Verbindung der allgemeinen Formel

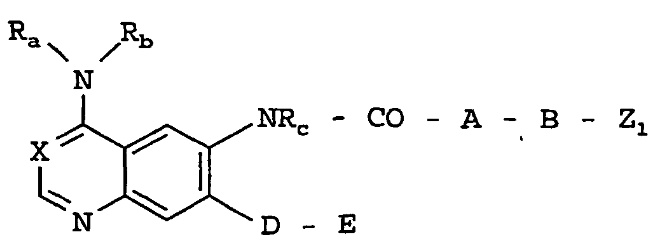

, (II)

in der

$R_a$ bis $R_c$, A, B, D, E und X wie in den Ansprüchen 1 bis 5 erwähnt definiert sind und
$Z_1$ eine Austrittsgruppe darstellt,

mit einer Verbindung der allgemeinen Formel

$$H - C , \qquad (III)$$

in der
C wie eingangs in den Ansprüchen 1 bis 5 definiert ist, umgesetzt wird oder
b) eine gegebenenfalls im Reaktionsgemisch gebildete Verbindung der allgemeinen Formel

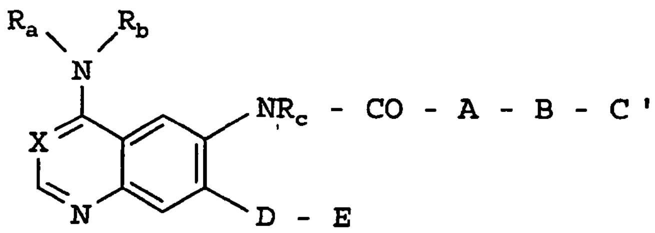

, (IV)

in der

$R_a$ bis $R_c$, A, B, D, E und X wie in den Ansprüchen 1 bis 5 erwähnt definiert sind und

C' eine durch Cyclisierung in eine Gruppe C überführbare, entsprechend substituierte N-(Carboxymethyl) -N-(2-hydroxyethyl)-amino- oder N-($C_{1-4}$-Alkyloxycarbonymethyl)-N-(2-hydroxyethyl)-aminogruppe bedeutet, cyclisiert wird und

erforderlichenfalls ein bei den vorstehend beschriebenen Umsetzungen verwendeter Schutzrest wieder abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträgliche Salze übergeführt wird.

## Claims

**1.** Bicyclic heterocycles of general formula

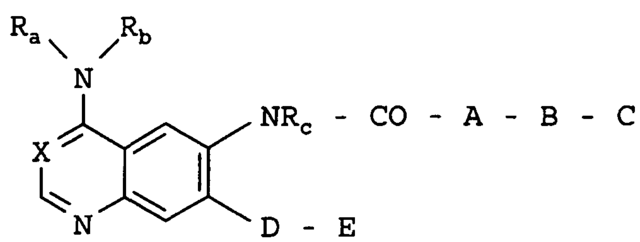

, (I)

wherein

$R_a$ denotes a hydrogen atom or a methyl group,

$R_b$ denotes a phenyl, benzyl- or 1-phenylethyl group wherein the phenyl nucleus is substituted in each case by the groups $R_1$ to $R_3$, while

$R_1$ and $R_2$, which may be identical or different, in each case denote a hydrogen, fluorine, chlorine, bromine or iodine atom,

a methyl, ethyl, hydroxy, methoxy, ethoxy, amino, cyano, vinyl or ethynyl group,

an aryl, aryloxy, arylmethyl or arylmethoxy group,

a methyl or methoxy group substituted by 1 to 3 fluorine atoms or

$R_1$ together with $R_2$, if they are bound to adjacent carbon atoms, denote a -CH=CH-CH=CH, -CH=CH-NH- or -CH=N-NH group and

$R_3$ denotes a hydrogen, fluorine, chlorine or bromine atom,

$R_c$ denotes a hydrogen atom or a methyl group,

X denotes a methyne group substituted by a cyano group or a nitrogen atom,

A denotes a 1,1- or 1,2-vinylene group which may be substituted in each case by one or two methyl groups or by a trifluoromethyl group,

an ethynylene group or

a 1,3-butadien-1,4-ylene group optionally substituted by a methyl or trifluoromethyl group,

B denotes an alkylene or -CO-alkylene group wherein the alkylene moiety in each case contains 1 to 4 carbon atoms, while the linking of the -CO-alkylene group to the adjacent group A in each case must take place via the carbonyl group,

a -CO-O-alkylene- or -CO-$NR_4$-alkylene group wherein the alkylene moiety in each case contains 1 to 4 carbon atoms, while the linking to the adjacent group A in each case must take place via the carbonyl group, wherein

$R_4$ denotes a hydrogen atom or a methyl or ethyl group,
or a carbonyl group,

C denotes a 2-oxo-morpholin-4-yl group substituted by the group $R_5$ or by the group $R_5$ and a $C_{1-4}$-alkyl group, while

$R_5$ denotes a $C_{3-4}$-alkyl, hydroxy-$C_{1-4}$-alkyl, $C_{1-4}$-alkoxy-$C_{1-4}$-alkyl, di- ($C_{1-4}$-alkyl)-amino-$C_{1-4}$-alkyl, pyrrolidino-$C_{1-4}$-alkyl, piperidino-$C_{1-4}$-alkyl, morpholino-$C_{1-4}$-alkyl, 4-($C_{1-4}$-alkyl)-piperazino-$C_{1-4}$-alkyl, $C_{1-4}$-alkylsulphanyl-$C_{1-4}$-alkyl, $C_{1-4}$-alkylsulphinyl-$C_{1-4}$-alkyl, $C_{1-4}$-alkylsulphonyl-$C_{1-4}$-alkyl, cyano-$C_{1-4}$-alkyl, $C_{1-4}$-alkoxycarbonyl-$C_{1-4}$-alkyl, aminocarbonyl-$C_{1-4}$-alkyl, $C_{1-4}$-alkyl-aminocarbonyl-$C_{1-4}$-alkyl, di-($C_{1-4}$-alkyl)aminocarbonyl-$C_{1-4}$-alkyl, pyrrolidinocarbonyl-$C_{1-4}$-alkyl, piperidinocarbonyl-$C_{1-4}$-alkyl, morpholinocarbonyl-$C_{1-4}$-alkyl or a 4-($C_{1-4}$-alkyl)-piperazinocarbonyl-$C_{1-4}$-alkyl group,

a 2-oxo-morpholin-4-yl group substituted by two groups $R_5$, where $R_5$ is as hereinbefore defined and the two groups $R_5$ may be identical or different,

a 2-oxo-morpholin-4-yl group, wherein the two hydrogen atoms of a methylene group are replaced by a $-(CH_2)_m$, $-CH_2-Y-CH_2$ , $-CH_2-Y-CH_2-CH_2$, $-CH_2CH_2-Y-CH_2CH_2-$ or $-CH_2CH_2-Y-CH_2CH_2CH_2-$ bridge optionally substituted by one or two $C_{1-2}$-alkyl groups, while

m denotes the number 2, 3, 4, 5 or 6 and
Y denotes an oxygen or sulphur atom, a sulphinyl, sulphonyl or $C_{1-4}$-alkylimino group,

a 2-oxo-morpholin-4-yl group, wherein a hydrogen atom in the 5 position together with a hydrogen atom in the 6 position is replaced by a $-(CH_2)_n$, $-CH_2-Y-CH_2$, $-CH_2-Y-CH_2CH_2-$ or $-CH_2CH_2-Y-CH_2$-bridge, while

Y is as hereinbefore defined and
n denotes the number 2, 3 or 4,

or, if D together with E denotes a group $R_d$, it may also denote a 2-oxo-morpholin-4-yl group which may be substituted by 1 to 4 $C_{1-2}$-alkyl groups,

D denotes a -O-$C_{1-6}$-alkylene group, while the alkylene moiety is linked to the group E, or

an oxygen atom, while this may not be linked to a nitrogen atom of the group E, and

E denotes an amino group substituted by 2 $C_{1-4}$-alkyl groups, wherein the alkyl groups may be identical or different and each alkyl moiety may be substituted from the 2 position by a $C_{1-4}$-alkoxy or di-($C_{1-4}$-alkyl)-amino group or by a 4- to 7-membered alkyleneimino group, while in the abovementioned 6-to 7-membered alkyleneimino groups in each case a methylene group may be replaced in the 4 position by an oxygen or sulphur atom or by a sulphinyl, sulphonyl- or N-($C_{1-4}$-alkyl)-imino group,

a 4- to 7-membered alkyleneimino group optionally substituted by 1 to 4 methyl groups,

a 6- to 7-membered alkyleneimino group optionally substituted by 1 or 2 methyl groups, wherein in each case a methylene group in the 4 position is replaced by an oxygen or sulphur atom or by a sulphinyl, sulphonyl- or N-($C_{1-4}$-alkyl)-imino group,

an imidazolyl group optionally substituted by 1 to 3 methyl groups,

a $C_{5-7}$-cycloalkyl group, wherein a methylene group is replaced by an oxygen or sulphur atom or by a sulphinyl, sulphonyl or N-($C_{1-4}$-alkyl)-imino group, or

D together with E denotes a hydrogen atom,

a $C_{1-6}$-alkoxy group optionally substituted from the 2 position by a hydroxy- or $C_{1-4}$-alkoxy group,

a $C_{3-7}$-cycloalkoxy- or $C_{3-7}$-cycloalkyl-$C_{1-4}$-alkoxy group,

or a group $R_d$, where

$R_d$ denotes a $C_{2-6}$-alkoxy group which is substituted from the 2 position by a $C_{4-7}$-cycloalkoxy- or $C_{3-7}$-cycloalkyl-$C_{1-3}$-alkoxy group,

a $C_{4-7}$-cycloalkoxy- or $C_{3-7}$-cycloalkyl-$C_{1-6}$-alkoxy group wherein the cycloalkyl moiety in each case is substituted by a $C_{1-4}$-alkyl, $C_{1-4}$-alkoxy, di-($C_{1-4}$-alkyl)-amino, pyrrolidino, piperidino, morpholino, piperazino, 4-($C_{1-2}$-alkyl)-piperazino, $C_{1-4}$-alkoxy-$C_{1-2}$-alkyl, di-($C_{1-4}$-alkyl)-amino-$C_{1-2}$-alkyl, pyrrolidino-$C_{1-2}$-alkyl, piperidino-$C_{1-2}$-alkyl, morpholino-$C_{1-2}$-alkyl, piperazino-$C_{1-2}$-alkyl- or 4-($C_{1-2}$-alkyl)-piperazino-$C_{1-2}$-alkyl group, while the abovementioned cycloalkyl moieties may additionally be substituted by a methyl or ethyl group,

while, unless otherwise stated, by the aryl moieties mentioned in the definition of the abovementioned groups is meant a phenyl group which may be mono- or disubstituted by $R_6$, while the substituents may be identical or different and

$R_6$ denotes a fluorine, chlorine, bromine or iodine atom, a $C_{1-2}$-alkyl, trifluoromethyl or $C_{1-2}$-alkoxy group, or

two groups $R_6$, if they are bound to adjacent carbon atoms, together represent a $C_{3-4}$-alkylene, methylenedioxy or 1,3-butadien-1,4-ylene group,

the tautomers, stereisomers and salts thereof.

**2.** Bicyclic heterocycles of general formula I according to claim 1, wherein

$R_a$ denotes a hydrogen atom,

$R_b$ denotes a benzyl or 1-phenylethyl group or a phenyl group substituted by the groups $R_1$ and $R_2$, while

$R_1$ denotes a hydrogen, fluorine, chlorine or bromine atom, a methyl, trifluoromethyl, cyano or ethynyl group and
$R_2$ denotes a hydrogen or fluorine atom,

$R_c$ denotes a hydrogen atom,

X denotes a nitrogen atom,

A denotes a 1,2-vinylene group,

B denotes a $C_{1-4}$-alkylene group,

C denotes a 2-oxo-morpholin-4-yl group substituted by the group $R_5$ or by the group $R_5$ and a $C_{1-4}$-alkyl group, while

> $R_5$ denotes a $C_{3-4}$-alkyl, $C_{1-2}$-alkoxy-$C_{1-4}$-alkyl, di-($C_{1-2}$-alkyl)-amino-$C_{1-4}$-alkyl, pyrrolidino-$C_{1-4}$-alkyl, piperidino-$C_{1-4}$-alkyl, morpholino-$C_{1-4}$-alkyl, 4-($C_{1-2}$-alkyl)-piperazino-$C_{1-4}$-alkyl, $C_{1-2}$-alkylsulphanyl-$C_{1-4}$-alkyl, $C_{1-2}$-alkylsulphinyl-$C_{1-4}$-alkyl, $C_{1-2}$-alkylsulphonyl-$C_{1-4}$-alkyl, cyano-$C_{1-4}$-alkyl, $C_{1-2}$-alkoxycarbonyl-$C_{1-4}$-alkyl, aminocarbonyl-$C_{1-4}$-alkyl, $C_{1-2}$-alkyl-aminocarbonyl-$C_{1-4}$-alkyl, di-($C_{1-2}$-alkyl)-aminocarbonyl-$C_{1-4}$-alkyl, pyrrolidinocarbonyl-$C_{1-4}$-alkyl, piperidinocarbonyl-$C_{1-4}$-alkyl, morpholinocarbonyl-$C_{1-4}$-alkyl-or a 4-($C_{1-2}$-alkyl)-piperazinocarbonyl-$C_{1-4}$-alkyl group,

a 2-oxo-morpholin-4-yl group substituted by two groups $R_5$, while $R_5$ is as hereinbefore defined and the two groups $R_5$ may be identical or different,

a 2-oxo-morpholin-4-yl group, wherein the two hydrogen atoms of a methylene group are replaced by a $(CH_2)_m$, $-CH_2-Y-CH_2$, $-CH_2-Y-CH_2-CH_2-$ or $-CH_2CH_2-Y-CH_2CH_2-$bridge, while

> m denotes the number 2, 3, 4 or 5 and
> Y denotes an oxygen or sulphur atom, a sulphinyl, sulphonyl or $C_{1-2}$-alkylimino group,

a 2-oxo-morpholin-4-yl group, wherein a hydrogen atom in the 5 position together with a hydrogen atom in the 6 position is replaced by a $-(CH_2)_n$, $-CH_2-Y-CH_2$, $-CH_2-Y-CH_2CH_2-$ or $-CH_2CH_2-Y-CH_2-$bridge, where

> Y is as hereinbefore defined and
> n denotes the number 2, 3 or 4,

or, if D together with E denotes a group $R_d$, it may also denote a 2-oxo-morpholin-4-yl group which may be substituted by 1 or 2 methyl or ethyl groups,

D denotes a $-O-C_{1-4}$-alkylene group, while the alkylene moiety is linked to the group E, and

E denotes a dimethylamino, diethylamino, pyrrolidino, piperidino, morpholino, 4-methyl-piperazino- or 4-ethylpiperazino group or

D together with E denotes a hydrogen atom,

a methoxy, ethoxy, 2-methoxy-ethoxy, 3-methoxy-propyloxy, tetrahydrofuran-3-yloxy, tetrahydropyran-3-yloxy, tetrahydropyran-4-yloxy, tetrahydrofuranylmethoxy or tetrahydropyranylmethoxy group,

a cyclobutyloxy, cyclopentyloxy, cyclohexyloxy, cyclopropylmethoxy, cyclobutylmethoxy, cyclopentylmethoxy or cyclohexylmethoxy group or

a group $R_d$, where

> $R_d$ denotes a 2-(cyclobutyloxy)-ethoxy, 2-(cyclopentyloxy)-ethoxy, 2-(cyclopropylmethoxy)-ethoxy or 2-(cyclobutylmethoxy)-ethoxy group,

the tautomers, stereoisomers and salts thereof.

**3.** Bicyclic heterocycles of general formula I according to claim 1, wherein

$R_a$ denotes a hydrogen atom,

$R_b$ denotes a 1-phenylethyl, 3-methylphenyl, 3-chlorophenyl, 3-bromophenyl- or 3-chloro-4-fluorophenyl group,

$R_c$ denotes a hydrogen atom,

X denotes a nitrogen atom,

A denotes a 1,2-vinylene group,

B denotes a methylene group,

C denotes a 2-oxo-morpholin-4-yl group which is substituted by a methoxymethyl, methoxyethyl, ethoxymethyl, ethoxyethyl, dimethylaminomethyl, dimethylaminoethyl, diethylaminomethyl, diethylaminoethyl, cyanomethyl or cyanoethyl group,

a 2-oxo-morpholin-4-yl group, wherein the two hydrogen atoms of a methylene group are replaced by a $-CH_2CH_2$, $-CH_2CH_2CH_2$, $-CH_2CH_2CH_2CH_2$, $-CH_2CH_2CH_2CH_2CH_2$, $-CH_2-O-CH_2CH_2$, $-CH_2-NCH_3-CH_2CH_2$, $-CH_2-NC_2H_5-CH_2CH_2$, $-CH_2CH_2-O-CH_2CH_2$, $-CH_2CH_2-NCH_3-CH_2CH_2-$ or $-CH_2CH_2-NC_2H_5-CH_2CH_2-$ bridge,

a 2-oxo-morpholin-4-yl group, wherein a hydrogen atom in the 5 position together with a hydrogen atom in the 6 position is replaced by a $-CH_2CH_2CH_2$, $-CH_2CH_2CH_2CH_2$, $-CH_2-O-CH_2$, $-CH_2-NCH_3-CH_2$, $-CH_2-NC_2H_5-CH_2$, $-CH_2-O-CH_2CH_2$, $-CH_2-NCH_3-CH_2CH_2$, $-CH_2-NC_2H_5-CH_2CH_2$, $-CH_2CH_2-O-CH_2$, $-CH_2CH_2-NCH_3-CH_2-$ or $-CH_2CH_2-NC_2H_5-CH_2-$ bridge,

or, if D together with E denotes a group $R_d$, it may also denote a 2-oxo-morpholin-4-yl group which is substituted by 1 or 2 methyl groups, and

D together with E denotes a hydrogen atom,

a methoxy, ethoxy, 2-methoxy-ethoxy, 3-methoxy-propyloxy, tetrahydrofuran-3-yloxy, tetrahydropyran-4-yloxy or tetrahydrofuranylmethoxy group,

a cyclobutyloxy, cyclopentyloxy, cyclopropylmethoxy, cyclobutylmethoxy or cyclopentylmethoxy group or

a group $R_d$, where

$R_d$ denotes a 2-(cyclobutyloxy)-ethoxy, 2-(cyclopentyloxy)-ethoxy, 2-(cyclopropylmethoxy)-ethoxy or 2-(cyclobutylmethoxy)-ethoxy group,

the tautomers, stereoisomers and salts thereof.

**4.** Bicyclic heterocycles of general formula I according to claim 1, wherein

$R_a$ denotes a hydrogen atom,

$R_b$ denotes a 3-chloro-4-fluorophenyl group,

$R_c$ denotes a hydrogen atom,

X denotes a nitrogen atom,

A denotes a 1,2-vinylene group,

B denotes a methylene group,

C denotes a 2-oxo-morpholin-4-yl group which is substituted by a methoxymethyl or methoxyethyl group, or

a 2-oxo-morpholin-4-yl group, wherein the two hydrogen atoms of a methylene group are replaced by a $-CH_2CH_2-O-CH_2CH_2-$ bridge, and

D together with E denotes a hydrogen atom, a methoxy or cyclopropylmethoxy group,

the tautomers, stereoisomers and salts thereof.

**5.** The following compounds of general formula I according to claim 1:

(1) 4-[(3-chloro-4-fluoro-phenyl)amino]-6-{[4-((R)-2-methoxymethyl-6-oxo-morpholin-4-yl)-1-oxo-2-buten-1-yl]amino)-7-cyclopropylmethoxy-quinazoline,

(2) 4-[(3-chloro-4-fluoro-phenyl)amino]-6-{[4-(2-oxo-1,9-dioxa-4-aza-spiro[5.5]undec-4-yl)-1-oxo-2-buten-1-yl]amino}-7-cyclopropylmethoxy-quinazoline and

(3) 4-[(3-chloro-4-fluoro-phenyl)amino]-6-({4-[2-(2-methoxyethyl)-6-oxo-morpholin-4-yl]-1-oxo-2-buten-1-yl} amino)-7-cyclopropylmethoxy-quinazoline,

the tautomers, stereisomers and salts thereof.

**6.** Physiologically acceptable salts of the compounds according to at least one of claims 1 to 5 with inorganic or organic acids or bases.

**7.** Pharmaceutical compositions containing a compound according to at least one of claims 1 to 5 or a physiologically acceptable salt according to claim 6 optionally together with one or more inert carriers and/or diluents.

**8.** Use of a compound according to at least one of claims 1 to 6 for preparing a pharmaceutical composition which is suitable for the treatment of benign or malignant tumours, for preventing and treating diseases of the respiratory tract and lungs, for treating polyps, diseases of the gastro-intestinal tract, bile duct and gall bladder as well as the kidneys and skin.

**9.** Process for preparing a pharmaceutical composition according to claim 7, **characterised in that** a compound according to at least one of claims 1 to 6 is incorporated in one or more inert carriers and/or diluents by a non-chemical method.

**10.** Process for preparing the compounds of general formula I according to claims 1 to 6, **characterised in that**

a) a compound of general formula

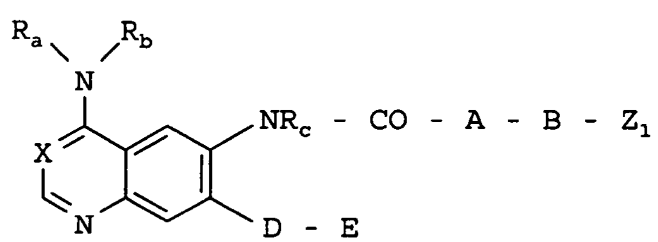

, (II)

optionally formed in a reaction mixture
wherein

$R_a$ to $R_c$, A, B, D, E and X are defined as in claims 1 to 5 and
$Z_1$ denotes a leaving group,

is reacted with a compound of general formula

H - C , (III)

wherein

C is defined as in claims 1 to 5 hereinbefore, or

b) a compound of general formula

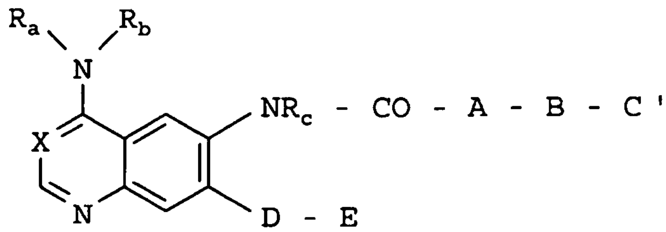

, (IV)

optionally formed in a reaction mixture
wherein

$R_a$ to $R_c$, A, B, D, E and X are defined as in claims 1 to 5 and C' denotes a correspondingly substituted N-(carboxymethyl)-N-(2-hydroxyethyl)-amino or N-($C_{1-4}$-alkyloxycarbonymethyl)-N-(2-hydroxyethyl)-amino group which can be converted into a group C by cyclising, is cyclised, and

if necessary any protecting group used in the reactions described above is cleaved again and/or

if desired a compound of general formula I thus obtained is resolved into its stereoisomers and/or

a compound of general formula I thus obtained is converted into the salts thereof, particularly, for pharmaceutical use, into the physiologically acceptable salts thereof.

**Revendications**

**1.** Hétérocycles bicycliques de formule générale

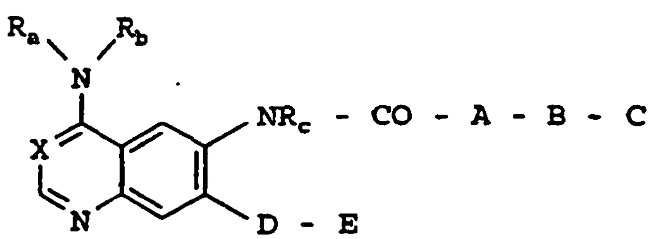

, (I)

où

$R_a$ représente un atome d'hydrogène ou un groupe méthyle,
$R_b$ représente un groupe phényle, benzyle ou 1-phényléthyle, dans lesquels le noyau phényle est substitué à chaque fois par les groupements $R_1$ à $R_3$, où

$R_1$ et $R_2$, qui peuvent être identiques ou différents, représentent à chaque fois un atome d'hydrogène, de fluor, de chlore, de brome ou d'iode,

un groupe méthyle, éthyle, hydroxyle, méthoxy, éthoxy, amino, cyano, vinyle ou éthynyle,
un groupe aryle, aryloxy, arylméthyle ou arylméthoxy,
un groupe méthyle ou méthoxy substitué par 1 à 3 atomes de fluor,
$R_1$ représente avec $R_2$, dans la mesure où ceux-ci sont liés à des atomes de carbone voisins, un groupe -CH=CH-CH=CH-, -CH=CH-NH- ou -CH=N-NH- et
$R_3$ représente un atome d'hydrogène, de fluor, de chlore ou de brome,

$R_c$ représente un atome d'hydrogène ou un groupe méthyle,
X représente un groupe méthyne substitué par un groupe cyano ou un atome d'azote,
A représente un groupe 1,1- ou 1,2-vinylène qui peut être substitué à chaque fois par un ou deux groupes méthyle ou par un groupe trifluorométhyle,
un groupe éthynylène ou
un groupe 1,3-butadién-1,4-ylène éventuellement substitué par un groupe méthyle ou trifluorométhyle,
B représente un groupe alkylène ou -CO-alkylène dans lesquels la partie alkylène contient à chaque fois 1 à 4 atomes de carbone, où la liaison du groupe -CO-alkylène au groupe A voisin doit se faire à chaque fois par le biais du groupe carbonyle,
un groupe -CO-O-alkylène ou -CO-$NR_4$-alkylène dans lesquels la partie alkylène contient à chaque fois 1 à 4 atomes de carbone, où la liaison avec le groupe A voisin doit se faire à chaque fois par le biais du groupe carbonyle, où

$R_4$ représente un atome d'hydrogène ou un groupe méthyle ou éthyle, ou un groupe carbonyle,

C représente un groupe 2-oxo-morpholin-4-yle substitué par le groupement $R_5$ ou par le groupement $R_5$ et un groupe $C_{1-4}$-alkyle, où

$R_5$ représente un groupe $C_{3-4}$-alkyle, hydroxy-$C_{1-4}$-alkyle, $C_{1-4}$-alcoxy-$C_{1-4}$-alkyle, di-($C_{1-4}$-alkyl)-amino-$C_{1-4}$-alkyle, pyrrolidino-$C_{1-4}$-alkyle, pipéridino-$C_{1-4}$-alkyle, morpholino-$C_{1-4}$-alkyle, 4-($C_{1-4}$-alkyl)-pipérazino-$C_{1-4}$-alkyle, $C_{1-4}$-alkylsulfanyl-$C_{1-4}$-alkyle, $C_{1-4}$-alkylsulfinyl-$C_{1-4}$-alkyle, $C_{1-4}$-alkylsulfonyl-$C_{1-4}$-alkyle, cyano-$C_{1-4}$-alkyle, $C_{1-4}$-alcoxycarbonyl-$C_{1-4}$-alkyle, amino-carbonyl-$C_{1-4}$-alkyle, $C_{1-4}$-alkyl-aminocarbonyl-$C_{1-4}$-alkyle, di-($C_{1-4}$-alkyl)-aminocarbonyl-$C_{1-4}$-alkyle, pyrrolidinocarbonyl-$C_{1-4}$-alkyle, pipéridino-carbonyl-$C_{1-4}$-alkyle, morpholinocarbonyl-$C_{1-4}$-alkyle ou 4-($C_{1-4}$-alkyl)-pipérazinocarbonyl-$C_{1-4}$-alkyle,

un groupe 2-oxo-morpholin-4-yle substitué par deux groupements $R_5$, où $R_5$ est défini comme indiqué précédemment et les deux groupements $R_5$ peuvent être identiques ou différents,
un groupe 2-oxo-morpholin-4-yle dans lequel les deux atomes d'hydrogène d'un groupe méthylène sont remplacés par un pont $-(CH_2)_m-$, $-CH_2-Y-CH_2-$, $-CH_2-Y-CH_2CH_2-$, $-CH_2CH_2-Y-CH_2CH_2-$ ou $-CH_2CH_2-Y-CH_2CH_2CH_2-$, où

m représente le nombre 2, 3, 4, 5 ou 6 et
Y représente un atome d'oxygène ou de soufre, un groupe sulfinyle, sulfonyle ou $C_{1-4}$-alkylimino,

un groupe 2-oxo-morpholin-4-yle dans lequel un atome d'hydrogène en position 5 est remplacé en même temps qu'un atome d'hydrogène en position 6 par un pont $-(CH_2)_n-$, $-CH_2-Y-CH_2-$, $-CH_2-Y-CH_2CH_2-$ ou $-CH_2CH_2-Y-CH_2-$, où

Y est défini comme indiqué précédemment et
n représente le nombre 2, 3 ou 4,

ou, si D représente avec E un groupe $R_d$, également un groupe 2-oxo-morpholin-4-yle qui peut être substitué par 1 à 4 groupes $C_{1-2}$-alkyle,
D représente un groupe -O-$C_{1-6}$-alkylène où la partie alkylène est liée au groupement E, ou
un atome d'oxygène, où celui-ci ne peut pas être lié à un atome d'azote du groupement E, et
E représente un groupe amino substitué par 2 groupes $C_{1-4}$-alkyle, dans lequel les groupements alkyle peuvent être identiques ou différents et chaque partie alkyle peut être substituée à partir de la position 2 par un groupe $C_{1-4}$-alcoxy, ou di-( $C_{1-4}$-alkyl)-amino ou par un groupe alkylèneimino à 4 à 7 chaînons, où, dans les groupes alkylèneimino à 6 à 7 chaînons cités précédemment, à chaque fois un groupe méthylène en position 4 peut être remplacé par un atome d'oxygène ou de soufre, par un groupe sulfinyle, sulfonyle ou N-( $C_{1-4}$-alkyl) -imino,

un groupe alkylèneimino à 4 à 7 chaînons éventuellement substitué par 1 à 4 groupes méthyle,
un groupe alkylèneimino à 6 à 7 chaînons éventuellement substitué par 1 ou 2 groupes méthyle où, à chaque fois, un groupe méthylène en position 4 est remplacé par un atome d'oxygène ou de soufre, par un groupe sulfinyle, sulfonyle ou N-($C_{1-4}$-alkyl) -imino,
un groupe imidazolyle éventuellement substitué par 1 à 3 groupes méthyle,
un groupe $C_{5-7}$-cycloalkyle dans lequel un groupe méthylène est remplacé par un atome d'oxygène ou de soufre, par un groupe sulfinyle, sulfonyle ou N-($C_{1-4}$-alkyl)-imino, ou
D représente avec E un atome d'hydrogène,
un groupe $C_{1-6}$-alcoxy éventuellement substitué à partir de la position 2 par un groupe hydroxyle ou $C_{1-4}$-alcoxy,
un groupe $C_{3-7}$-cycloalcoxy ou $C_{3-7}$-cycloalkyl-$C_{1-4}$-alcoxy,
ou un groupement $R_d$, où

$R_d$ représente un groupe $C_{2-6}$-alcoxy qui est substitué à partir de la position 2 par un groupe $C_{4-7}$-cycloalcoxy ou $C_{3-7}$-cycloalkyl-$C_{1-3}$-alcoxy,
un groupe $C_{4-7}$-cycloalcoxy ou $C_{3-7}$-cycloalkyl-$C_{1-6}$-alcoxy dans lesquels la partie cycloalkyle est substituée à chaque fois par un groupe $C_{1-4}$-alkyle, $C_{1-4}$-alcoxy, di-($C_{1-4}$-alkyl)-amino, pyrrolidino, pipéridino, morpholino, pipérazino, 4-($C_{1-2}$-alkyl)-pipérazino, $C_{1-4}$-alcoxy-$C_{1-2}$-alkyle, di-($C_{1-4}$-alkyl)-amino-$C_{1-2}$-alkyle, pyrrolidino-$C_{1-2}$-alkyle, pipéridino-$C_{1-2}$-alkyle, morpholino-$C_{1-2}$-alkyle, pipérazino-$C_{1-2}$-alkyle ou 4-($C_{1-2}$-alkyl)-pipérazino-$C_{1-2}$-alkyle, où les parties cycloalkyle citées précédemment peuvent être substituées en outre par un groupe méthyle ou éthyle,
où, sauf indication contraire, par les parties aryle citées lors de la définition des groupements cités précédemment, on doit comprendre un groupe phényle qui peut être mono- ou disubstitué par $R_6$, où les substituants peuvent être identiques ou différents et

$R_6$ représente un atome de fluor, de chlore, de brome ou d'iode, un groupe $C_{1-2}$-alkyle, trifluorométhyle ou $C_{1-2}$-alcoxy, ou
deux groupements $R_6$, dans la mesure où ils sont liés à des atomes de carbone voisins, représentent ensemble un groupe $C_{3-4}$-alkylène, méthylènedioxy ou 1,3-butadién-1,4-ylène,

leurs tautomères, leurs stéréoisomères et leurs sels.

**2.** Hétérocycles bicycliques de formule générale I selon la revendication 1, où

$R_a$ représente un atome d'hydrogène,
$R_b$ représente un groupe benzyle ou 1-phényléthyle, ou un groupe phényle substitué par les groupements $R_1$ et $R_2$, où

$R_1$ représente un atome d'hydrogène, de fluor, de chlore ou de brome, un groupe méthyle, trifluorométhyle, cyano ou éthynyle et
$R_2$ représente un atome d'hydrogène ou un atome de fluor,

$R_c$ représente un atome d'hydrogène,
X représente un atome d'azote,
A représente un groupe 1,2-vinylène,
B représente un groupe $C_{1-4}$-alkylène,
C représente un groupe 2-oxo-morpholin-4-yle substitué par le groupement $R_5$ ou par le groupement $R_5$ et un groupe $C_{1-4}$-alkyle, où

$R_5$ représente un groupe $C_{3-4}$-alkyle, $C_{1-2}$-alcoxy-$C_{1-4}$-alkyle, di-($C_{1-2}$-alkyl)-amino-$C_{1-4}$-alkyle, pyrrolidino-$C_{1-4}$-alkyle, pipéridino-$C_{1-4}$-alkyle, morpholino-$C_{1-4}$-alkyle, 4-($C_{1-2}$-alkyl)-pipérazino-$C_{1-4}$-alkyle, $C_{1-2}$-alkylsulfanyl-$C_{1-4}$-alkyle, $C_{1-2}$-alkylsulfinyl-$C_{1-4}$-alkyle, $C_{1-2}$-alkylsulfonyl-$C_{1-4}$-alkyle, cyano-$C_{1-4}$-alkyle, $C_{1-2}$-alcoxycarbonyl-$C_{1-4}$-alkyle, aminocarbonyl-$C_{1-4}$-alkyle, $C_{1-2}$-alkyl-aminocarbonyl-$C_{1-4}$-alkyle, di-($C_{1-2}$-alkyl)-aminocarbonyl-$C_{1-4}$-alkyle, pyrrolidinocarbonyl-$C_{1-4}$-alkyle, pipéridinocarbonyl-$C_{1-4}$-alkyle, morpholinocarbonyl-$C_{1-4}$-alkyle ou 4-($C_{1-2}$-alkyl)-pipérazinocarbonyl-$C_{1-4}$-alkyle,

un groupe 2-oxo-morpholin-4-yle substitué par deux groupement $R_5$, où $R_5$ est défim comme indiqué précédemment et les deux groupements $R_5$ peuvent être identiques ou différents, un groupe 2-oxo-morpholin-4-yle

dans lequel les deux atomes d'hydrogène d'un groupe méthylène sont remplacés par un pont $-(CH_2)_m-$, $-CH_2-Y-CH_2-$, $-CH_2-Y-CH_2CH_2-$ ou $-CH_2CH_2-Y-CH_2CH_2-$, où

m représente le nombre 2, 3, 4 ou 5 et
Y représente un atome d'oxygène ou de soufre, un groupe sulfinyle, sulfonyle ou $C_{1-2}$-alkylimino,

un groupe 2-oxo-morpholin-4-yle dans lequel un atome d'hydrogène en position 5 est remplacé en même temps qu'un atome d'hydrogène en position 6 par un pont $-(CH_2)_n-$, $-CH_2-Y-CH_2-$, $-CH_2-Y-CH_2CH_2-$ ou $-CH_2CH_2-Y-CH_2-$, où

Y est défini comme indiqué précédemment et
n représente le nombre 2, 3 ou 4, ou, si D représente avec E un groupe $R_d$, également un groupe 2-oxo-morpholin-4-yle qui peut être substitué par 1 ou 2 groupes méthyle ou éthyle,

D représente un groupe $-O-C_{1-4}$-alkylène où la partie alkylène est liée au groupement E, et
E représente un groupe diméthylamino, diéthylamino, pyrrolidino, pipéridino, morpholino, 4-méthyl-pipérazino ou 4-éthyl-pipérazino ou
D représente avec E un atome d'hydrogène,
un groupe méthoxy, éthoxy, 2-méthoxy-éthoxy, 3-méthoxy-propyloxy, tétrahydrofuran-3-yloxy, tétrahydropyran-3-yloxy, tétrahydropyran-4-yloxy, tétrahydrofuranylméthoxy ou tétrahydropyranylméthoxy,
un groupe cyclobutyloxy, cyclopentyloxy, cyclohexyloxy, cyclopropylméthoxy, cyclobutylméthoxy, cyclopentylméthoxy ou cyclohexylméthoxy ou
un groupement $R_d$, où

$R_d$ représente un groupe 2-(cyclobutyloxy)-éthoxy, 2-(cyclopentyloxy)-éthoxy, 2-(cyclopropylméthoxy)-éthoxy ou 2-(cyclobutylméthoxy)-éthoxy,

leurs tautomères, leurs stéréoisomères et leurs sels.

**3.** Hétérocycles bicycliques de formule générale I selon la revendication 1, où

$R_a$ représente un atome d'hydrogène,
$R_b$ représente un groupe 1-phényléthyle, 3-méthylphényle, 3-chlorophényle, 3-bromophényle ou 3-chloro-4-fluorophényle,
$R_c$ représente un atome d'hydrogène,
X représente un atome d'azote,
A représente un groupe 1,2-vinylène,
B représente un groupe méthylène,
C représente un groupe 2-oxo-morpholin-4-yle qui est substitué par un groupe méthoxyméthyle, méthoxyéthyle, éthoxyméthyle, éthoxyéthyle, diméthylaminométhyle, diméthylaminoéthyle, diéthylaminométhyle, diéthylaminoéthyle, cyanométhyle ou cyanoéthyle,
un groupe 2-oxo-morpholin-4-yle dans lequel les deux atomes d'hydrogène d'un groupe méthylène sont remplacés par un pont $-CH_2CH_2-$, $-CH_2CH_2CH_2-$, $-CH_2CH_2CH_2CH_2-$, $-CH_2CH_2CH_2CH_2CH_2-$, $-CH_2-O-CH_2CH_2-$, $-CH_2-NCH_3-CH_2CH_2-$, $-CH_2-NC_2H_5-CH_2CH_2-$, $-CH_2CH_2-O-CH_2CH_2-$, $-CH_2CH_2-NCH_3-CH_2CH_2-$ ou $-CH_2CH_2-NC_2H_5-CH_2CH_2-$,
un groupe 2-oxo-morpholin-4-yle dans lequel un atome d'hydrogène en position 5 est remplacé en même temps qu'un atome d'hydrogène en position 6 par un pont $-CH_2CH_2CH_2-$, $-CH_2CH_2CH_2CH_2-$, $-CH_2-O-CH_2-$, $-CH_2-NCH_3-CH_2-$, $-CH_2-NC_2H_5-CH_2-$, $-CH_2-O-CH_2CH_2-$, $-CH_2-NCH_3-CH_2CH_2-$, $-CH_2-NC_2H_5-CH_2CH_2-$, $-CH_2CH_2-O-CH_2-$, -CH2CH2-$NCH_3-CH_2-$ ou $-CH_2CH_2-NC_2H_5-CH_2-$,
ou, si D représente avec E un groupe $R_d$, également un groupe 2-oxo-morpholin-4-yle qui est substitué par 1 ou 2 groupes méthyle, et
D représente avec E un atome d'hydrogène,
un groupe méthoxy, éthoxy, 2-méthoxy-éthoxy, 3-méthoxy-propyloxy, tétrahydrofuran-3-yloxy, tétrahydropyran-4-yloxy ou tétrahydrofuranylméthoxy,
un groupe cyclobutyloxy, cyclopentyloxy, cyclopropylméthoxy, cyclobutylméthoxy ou cyclopentylméthoxy ou ou un groupement $R_d$, où
$R_d$ représente un groupe 2-(cyclobutyloxy)-éthoxy, 2-(cyclopentyloxy)-éthoxy, 2-(cyclopropylméthoxy)-éthoxy ou 2-(cyclobutylméthoxy)-éthoxy,

leurs tautomères, leurs stéréoisomères et leurs sels.

**4.** Hétérocycles bicycliques de formule générale I selon la revendication 1, où

$R_a$ représente un atome d'hydrogène,
$R_b$ représente un groupe 3-chloro-4-fluorophényle,
$R_c$ représente un atome d'hydrogène,
X représente un atome d'azote,
A représente un groupe 1,2-vinylène,
B représente un groupe méthylène,
C représente un groupe 2-oxo-morpholin-4-yle qui est substitué par un groupe méthoxyméthyle ou méthoxyéthyle, ou
un groupe 2-oxo-morpholin-4-yle dans lequel les deux atomes d'hydrogène d'un groupe méthylène sont remplacés par un pont $-CH_2CH_2-O-CH_2CH_2-$, et
D représente avec E un atome d'hydrogène, un groupe méthoxy ou cyclopropylméthoxy,

leurs tautomères, leurs stéréoisomères et leurs sels.

**5.** Composés de formule générale 1 selon la revendication 1 suivants :

(1) 4-[(3-chloro-4-fluoro-phényl)amino]-6-{[4-((*R*)-2-méthoxy-méthyl-6-oxo-morpholin-4-yl)-1-oxo-2-butén-1-yl]amino}-7-cyclopropylméthoxy-quinazoline,
(2) 4-[(3-chloro-4-fluoro-phényl)amino]-6-{[4-(-2-oxo-1,9-dioxa-4-azaspiro[5.5]-undéc-4-yl)-1-oxo-2-butén-1-yl]amino}-7-cyclopropylméthoxyquinazoline et
(3) 4-[(3-chloro-4-fluoro-phényl)amino]-6-({4-[2-(2-méthoxy-éthyl)-6-oxo-morpholin-4-yl]-1-oxo-2-butén-1-yl} amino)-7-cyclopropylméthoxy-quinazoline,

leurs tautomères, leurs stéréoisomères et leurs sels.

**6.** Sels physiologiquement acceptables des composés selon au moins l'une des revendications 1 à 5 avec des acides ou des bases inorganiques ou organiques.

**7.** Médicament contenant un composé selon au moins l'une des revendications 1 à 5 ou un sel physiologiquement acceptable selon la revendication 6 outre éventuellement un ou plusieurs supports et/ou diluants inertes.

**8.** Utilisation d'un composé selon au moins l'une des revendications 1 à 6 pour la fabrication d'un médicament qui convient pour le traitement des tumeurs bénignes ou malignes, pour la prévention et le traitement des maladies des voies respiratoires et des poumons, pour le traitement des polypes, des maladies des voies gastro-intestinales, des voies biliaires et de la vésicule biliaire ainsi que des reins et de la peau.

**9.** Procédé de fabrication d'un médicament selon la revendication 7 **caractérisé en ce que**, par voie non chimique, un composé selon au moins l'une des revendications 1 à 6 est incorporé dans un ou plusieurs supports et/ou diluants inertes.

**10.** Procédé de préparation des composés de formule générale I selon les revendications 1 à 6 **caractérisé en ce que**

a) un composé éventuellement formé dans le mélange réactionnel de formule générale

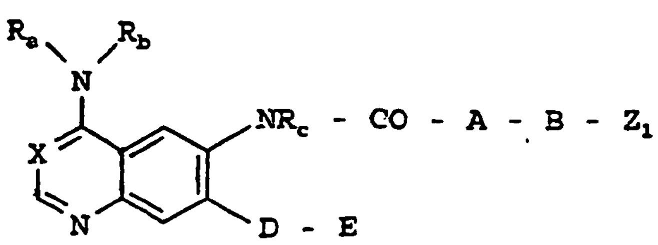

, (II)

où

$R_a$ à $R_c$, A, B, D, E et X sont définis comme indiqué dans les revendications 1 à 5 et
$Z_1$ représente un groupe partant,
est mis à réagir avec un composé de formule générale

H - C (III)

où

C est défini comme au début dans les revendications 1 à 5, ou
b) un composé éventuellement formé dans le mélange réactionnel de formule générale

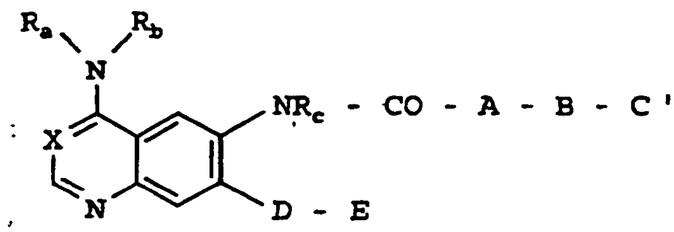

, (IV)

où

$R_a$ à $R_c$, A, B, D, E et X sont définis comme indiqué dans les revendications 1 à 5 et
C' représente un groupe N-(carboxyméthyl)-N-(2-hydroxyéthyl)-amino ou N-($C_{1-4}$-alkyloxycarbonylméthyl)-N-(2-hydroxyéthyl)-amino, substitué de manière correspondante, pouvant être converti par cyclisation en un groupe C, est cyclisé et
si nécessaire, un groupement protecteur utilisé dans les réactions décrites précédemment est clivé et/ou si on le souhaite, un composé de formule générale I ainsi obtenu est résolu en ses stéréoisomères et/ou un composé de formule générale I ainsi obtenu est converti en ses sels, en particulier pour l'utilisation pharmaceutique en ses sels physiologiquement acceptables.